# EUROPEAN PATENT APPLICATION

(11) **EP 3 838 281 A1**
(43) Date of publication of application: **23.06.2021**
(21) Application number: 19219112.0
(22) Date of filing: 20.12.2019
(51) Int. Cl.: A61K 35/74, A61K 9/00, A61K 9/19, A61P 25/00, A61P 25/18, A61P 25/24, A61P 25/28

(54) **COMPOSITIONS COMPRISING BACTERIAL STRAINS**

(71) Applicant: 4D Pharma Research Limited, Aberdeen, Aberdeenshire AB25 2ZS (GB)
(72) Inventor: The designation of the inventor has not yet been filed
(74) Representative: Brand Murray Fuller LLP

(57) **Abstract**

The invention provides compositions comprising bacterial strains and the use of such compositions in the treatment of disease, in particular central nervous system disorders.

SEQUENCE LISTING

<110>   4D Pharma Research Limited

<120>   COMPOSITIONS COMPRISING BACTERIAL STRAINS

<130>   P077074EP

<141>   2019-12-20

<160>   1

<170>   SeqWin2010, version 1.0

<210>   1
<211>   1399
<212>   DNA
<213>   Clostridiaceae

<400>   1
cttcggccgc gcccctttgc agttaggcta cggacttcgg gtgttcccga ctctcatggt  60
gtgacgggcg gtgtgtacaa ggcccgggaa cgtattcacc gcggcatgct gatccgcgat  120
tactagcgat tccgacttca tacaggcggg tttcagcctg caatccgaac tgggacggct  180
tttaggggtt tgctctgcct cgcggctttg cctccctctg ttaaccgcca ttgtagtacg  240
tgtgtggccc aggacataag gggcatgatg atttgacgtc gtccccacct tcctccgttt  300
tgtcaacggc agtctcgcta gagtgctctt gcgtagcaac taacaataag ggttgcgctc  360
gttgcgggac ttaacccaac atctcacgac acgagctgac gacaaccatg caccacctgt  420
ctctactttc cccgaagggc acctaatcca tctctggttc gttagtagga tgtcaagccc  480
tggtaaggtt cttcgcgttg cttcgaatta aaccacatac tccaccgctt gtgcgggccc  540
ccgtcaattc ctttgagttt caaccttgcg atcgtactcc ccaggtggga tacttattgc  600
gttagctgcg gcacggaggg ggtcagaccc cccacaccta gtatccatcg tttacggcgt  660
ggactaccag ggtatctaat cctgtttgct ccccacgctt tcgcgcctca gcgtcagtta  720
tcgtccagca atccgccttc gccactggtg ttcctccgta tatctacgca tttcaccgct  780
acacacggaa ttccgattgc ctctccgaca ctcaagaact acagtttcaa atgcaggcta  840
tgggttgagc ccatagtttt cacatctgac ttgcagtccc gcctacacgc cctttacacc  900
cagtaaatcc ggataacgct tgccacctac gtattaccgc ggctgctggc acgtagttag  960
ccgtggctta ttcaacaggt accgtcactt gcttcgtccc tgttaaaaga agtttacaac  1020
ccgaaagcct tcttccttca cgcggcgttg ctgggtcagg cttgcgccca ttgcccaata  1080
ttccccactg ctgcctcccg taggagtctg ggccgtgtct cagtcccaat gtggccggcc  1140
aacctctcag tccggctact gatcgtcgac ttggtgggcc gttaccccgc caactatcta  1200
atcagacgcg agtccatctc agagcgataa atctttgata tcagtgccat gcgacaccaa  1260
tatgtcatgc ggtattagca cagttttcac tgtgttattc cccactccaa ggcaggttac  1320
tcacgcgtta ctcacccgtc cgccactaaa cagccaattc cattgtccga aaactctttc  1380
cttggcgtcc gtcgactgc                                                1399

## Description

### TECHNICAL FIELD

This invention is in the field of compositions comprising bacterial strains and the use of such compositions in the treatment of disease.

### BACKGROUND TO THE INVENTION

The human intestine is thought to be sterile *in utero,* but it is exposed to a large variety of maternal and environmental microbes immediately after birth. Thereafter, a dynamic period of microbial colonization and succession occurs, which is influenced by factors such as delivery mode, environment, diet and host genotype, all of which impact upon the composition of the gut microbiota, particularly during early life. Subsequently, the microbiota stabilizes and becomes adult-like [1]. The human gut microbiota contains more than 500-1000 different phylotypes belonging essentially to two major bacterial divisions, the Bacteroidetes and the Firmicutes [2]. The successful symbiotic relationships arising from bacterial colonization of the human gut have yielded a wide variety of metabolic, structural, protective and other beneficial functions. The enhanced metabolic activities of the colonized gut ensure that otherwise indigestible dietary components are degraded with release of by-products providing an important nutrient source for the host. Similarly, the immunological importance of the gut microbiota is well-recognized and is exemplified in germfree animals which have an impaired immune system that is functionally reconstituted following the introduction of commensal bacteria [3-5].

The discovery of the size and complexity of the human microbiome has resulted in an on-going evaluation of many concepts of health and disease. Certainly, dramatic changes in microbiota composition have been documented in gastrointestinal disorders such as inflammatory bowel disease (IBD)[6-9]. More recently, there is increased interest in the art regarding alternations in the gut microbiome that may play a pathophysiological role in human brain diseases [10]. Preclinical and clinical evidence are strongly suggesting a link between brain development and microbiota [11].

A growing body of preclinical literature has demonstrated bidirectional signalling between the brain and the gut microbiome, involving multiple neurocrine and endocrine signalling systems. Indeed, increased levels of *Clostridium* species in the microbiome have been linked to brain disorders [12], and an imbalance of the Bacteroidetes and Firmicutes phyla has also been implicated in brain development disorders [13]. Suggestions that altered levels of gut commensals, including those of Bifidobacterium, Lactobacillus, Sutterella, Prevotella and Ruminococcus genera and of the Alcaligenaceae family are involved in immune-mediated central nervous system (CNS) disorders, are questioned by studies suggesting a lack of alteration in the microbiota between patients and healthy subjects [14]. This indicates that, at present, the practical effect of the link between the microbiome and human brain diseases is poorly characterised. Accordingly, more direct analytical studies are required to identify the therapeutic impact of altering the microbiome on CNS disorders.

In recognition of the potential positive effect that certain bacterial strains may have on the animal gut, various strains have been proposed for use in the treatment of various diseases (see, for example, [14-17]). Also, certain strains, including mostly *Lactobacillus* and *Bifidobacterium* strains, have been proposed for use in treating various inflammatory and autoimmune diseases that are not directly linked to the intestines (see [18] and [19] for reviews). In addition, a range of probiotics have been investigated in animal models to determine a role of the gut microbiome in modulating emotional behaviour, and *Bifidobacterium* and *Lactobacillus* are the main genera showing beneficial effects, reducing anxiety and repetitive behaviours, and increasing social interaction [20-22]. However, the relationship between different diseases and different bacterial strains, and the precise effects of particular bacterial strains on the gut and at a systemic level and on any particular types of diseases, are poorly characterised, particularly for central nervous system diseases.

There is a growing body of evidence to suggest that the microbiota-gut-brain axis is affected in autism spectrum disorders (ASD) and other neurodevelopmental and neuropsychiatric disorders. Animal models have provided considerable insight into how the microbiota may be involved in ASD. Furthermore, preclinical studies have demonstrated that targeting the gut microbiota through administration of beneficial live biotherapeutics effects an improvement in autistic-related behaviour in animal models, including the maternal immune activation (MIA) mouse model and the black and tan, brachyuric (BTBR) mouse. The BTBR mouse is a genetically modified, inbred mouse strain that displays a number of behaviours associated with ASD such as impaired sociability, repetitive behaviour and increased anxiety. Moreover, these mice also exhibit gastrointestinal dysfunctions along with alterations to the composition of the gut microbiota. Consequently, it represents an appropriate animal model for investigating the role of the microbiota-gut-brain axis in ASD.

There is a requirement in the art for new methods of treating central nervous system disorders. There is also a requirement for the potential effects of gut bacteria to be characterised so that new therapies using gut bacteria can be developed.

### SUMMARY OF THE INVENTION

The inventors have developed new therapies. In particular, the inventors have identified new therapies for treating and preventing central nervous system disorders. In particular, the inventors have developed new therapies for treating and preventing central nervous system disorders and conditions, in particular those mediated by the microbiota-gut-brain axis. The inventors have identified that bacterial strains of the family *Clostridiaceae* can be effective for treating and preventing diseases and conditions mediated by the microbiota-gut-brain axis. As described in the examples, administration (in particular oral administration) of compositions comprising an *Clostridiaceae* strain may reduce symptoms associated with dysfunction of the microbiota-gut-brain axis in a mouse model of autism spectrum disorders. In addition, as described in the examples, administration (in particular oral administration) of compositions comprising a *Clostridiaceae* strain may modulate the levels of signalling molecules associated with the function of the microbiota-gut-brain axis, and neurodevelopmental and neuropsychiatric disorders.

In some embodiments, the invention provides a composition comprising a bacterial strain of the family *Clostridiaceae,* for use in a method of treating or preventing a neurodevelopmental disorder or a neuropsychiatric condition. In certain embodiments, the invention provides a composition comprising the bacterial strain deposited under accession number NCIMB 43454 for use in a method of treating or preventing a neurodevelopmental disorder or neuropsychiatric condition.

In particular embodiments, the invention provides a composition comprising a bacterial strain of the family *Clostridiaceae,* for use in a method of treating or preventing a disease or condition selected from the group consisting of: autism spectrum disorders (ASDs); child developmental disorder; obsessive compulsive disorder (OCD); major depressive disorder; depression; seasonal affective disorder; anxiety disorders; chronic fatigue syndrome (myalgic encephalomyelitis); stress disorder; post-traumatic stress disorder; schizophrenia spectrum disorders; schizophrenia; bipolar disorder; psychosis; mood disorder; dementia; Alzheimer's; Parkinson's disease; and/or chronic pain. The data obtained by the inventors suggest that bacterial strains of the family *Clostridiaceae* are useful for treatment of these diseases, as demonstrated in the Examples. In further embodiments, the compositions of the invention may be useful for treating or preventing multiple sclerosis; motor neuron disease; Huntington's disease; Guillain-Barré syndrome and/or meningitis. The effect shown for the bacterial strains from the family *Clostridiaceae* on the microbiota-gut-brain axis and on diseases mediated by the microbiota-gut-brain axis may provide therapeutic benefits for other diseases and conditions mediated by the microbiota-gut-brain axis, such as those listed above.

In other embodiments, the invention provides a composition comprising a bacterial strain of the family *Clostridiaceae,* for use in a method of treating comorbidities associated with diseases and conditions mediated by the microbiota-gut-brain axis, such as those listed above. In particularly preferred embodiments, the invention provides a composition comprising a bacterial strain of the family *Clostridiaceae,* for use in a method of treating gastrointestinal comorbidities associated with diseases and conditions mediated by the microbiota-gut-brain axis, such as those listed above. The mouse model experiments used in this application for the assessment of the symptoms of autism spectrum disorders are known in the art to be applicable for the assessment of the symptoms of other central nervous system disorders including those listed above [23-25].

In particularly preferred embodiments, the invention provides a composition comprising a bacterial strain of the family *Clostridiaceae,* for use in a method of treating or preventing autism spectrum disorders, such as autism. The inventors have identified that treatment with *Clostridiaceae* strains can reduce symptom severity in a mouse model of autism spectrum disorders and can prevent or reduce stereotyped, repetitive and compulsive behaviour. In preferred embodiments, the invention provides a composition comprising a bacterial strain of the family *Clostridiaceae,* for use in the treatment of the behavioural and/or gastrointestinal symptoms of autism spectrum disorders. Treatment with *Clostridiaceae* strains may modulate signalling in the central, autonomic and enteric nervous systems; may modulate the activity of the HPA axis pathway; may modulate neuroendocrine and/or neuroimmune pathways; and/or may modulate the levels of commensal metabolites, inflammatory markers and/or gastrointestinal permeability of a subject, all of which are implicated in the neuropathology of autism spectrum disorders. In certain embodiments, treatment with *Clostridiaceae* may modulate the levels of oxytocin and/or vasopressin hormones. In preferred embodiments, the invention provides a composition comprising the strain deposited under accession number NCIMB 43454 for use in a method of treating or preventing autism spectrum disorders.

In further preferred embodiments, the invention provides a composition comprising a bacterial strain of the family *Clostridiaceae,* for use in a method of treating or preventing obsessive compulsive disorder (OCD). In preferred embodiments, the invention provides a composition for use in reducing stereotyped, repetitive, compulsive or anxious behaviour in the treatment of OCD. Treatment with *Clostridiaceae* may modulate signalling in the central, autonomic and enteric nervous systems; may modulate the activity of the HPA axis pathway; may modulate neuroendocrine and/or neuroimmune pathways; and/or may modulate the levels of commensal metabolites and/or gastrointestinal permeability of a subject, all of which are implicated in the neuropathology of OCD. In preferred embodiments, the invention provides a composition comprising the strain deposited under accession number NCIMB 43454 for use in a method of treating or preventing OCD.

In further preferred embodiments, the invention provides a composition comprising a bacterial strain of the family *Clostridiaceae,* for use in a method of treating or preventing major depressive disorder (MDD). As shown in the examples, treatment with *Clostridiaceae* strains reduced signs of depression in a mouse model. In preferred embodiments, the invention provides a composition comprising a bacterial strain of the family *Clostridiaceae,* for use in the treatment of depression. In preferred embodiments, the invention provides a composition for use in reducing stereotyped, repetitive, compulsive or anxious behaviour in the treatment of depression. Treatment with *Clostridiaceae* strains may modulate signalling in the central, autonomic and enteric nervous systems; may modulate the activity of the HPA axis pathway; may modulate neuroendocrine and/or neuroimmune pathways; and may modulate the levels of commensal metabolites, inflammatory markers and/or gastrointestinal permeability of a subject, all of which are implicated in the neuropathology of MDD. In certain embodiments, treatment with *Clostridiaceae* strains may modulate the levels of oxytocin and/or vasopressin hormones. In preferred embodiments, the invention provides a composition comprising the strain deposited under accession number NCIMB 43454 for use in a method of treating or preventing MDD.

In further preferred embodiments, the invention provides a composition comprising a bacterial strain of the family *Clostridiaceae,* for use in a method of treating or preventing anxiety disorders. In preferred embodiments, the invention provides a composition comprising a bacterial strain of the family *Clostridiaceae,* for use in the treatment of anxiety disorder. Treatment with *Clostridiaceae* may modulate signalling in the central, autonomic and enteric nervous systems; may modulate the activity of the HPA axis pathway; may modulate neuroendocrine and/or neuroimmune pathways; and may modulate the levels of commensal metabolites, inflammatory markers and/or gastrointestinal permeability of a subject, all of which are implicated in the neuropathology of anxiety disorders. In preferred embodiments, the invention provides a composition for use in reducing stereotyped, repetitive, compulsive or anxious behaviour in the treatment of anxiety. In preferred embodiments, the invention provides a composition comprising the strain deposited under accession number NCIMB 43454 for use in a method of treating or preventing anxiety disorders.

In further preferred embodiments, the invention provides a composition comprising a bacterial strain of the family *Clostridiaceae,* for use in a method of treating or preventing stress disorders, such as post-traumatic stress disorder. Compositions comprising a bacterial strain of the family *Clostridiaceae* may reduce stress in mouse models of stress disorders. Treatment with *Clostridiaceae* strains may modulate signalling in the central, autonomic and enteric nervous systems; may modulate the activity of the HPA axis pathway; may modulate neuroendocrine and/or neuroimmune pathways; and may modulate the levels of commensal metabolites, inflammatory markers and/or gastrointestinal permeability of a subject, all of which are implicated in the neuropathology of stress disorder. In certain embodiments, treatment with *Clostridiaceae* strains may modulate the levels of oxytocin and/or vasopressin hormones. In preferred embodiments, the invention provides a composition comprising the strain deposited under accession number NCIMB 43454 for use in a method of treating or preventing stress disorders.

In further preferred embodiments, the invention provides a composition comprising a bacterial strain of the family *Clostridiaceae,* for use in a method of treating or preventing schizophrenia spectrum and psychotic disorders, such as schizophrenia. Compositions comprising a bacterial strain of the family *Clostridiaceae* may improve positive and negative symptoms in mouse models of schizophrenia spectrum and psychotic disorders. Treatment with *Clostridiaceae* strains may modulate signalling in the central, autonomic and enteric nervous systems; may modulate the activity of the HPA axis pathway; may modulate neuroendocrine and/or neuroimmune pathways; and may modulate the levels of commensal metabolites and/or gastrointestinal permeability of a subject, all of which are implicated in the neuropathology of schizophrenia spectrum and psychotic disorders. In preferred embodiments, the invention provides a composition comprising the strain deposited under accession number NCIMB 43454 for use in a method of treating or preventing schizophrenia spectrum and psychotic disorders.

In further preferred embodiments, the invention provides a composition comprising a bacterial strain of the family *Clostridiaceae,* for use in a method of treating or preventing bipolar disorder. Compositions comprising a bacterial strain of the family *Clostridiaceae* may reduce occasions of mania and/or depression in mouse models of bipolar disorder. Treatment with *Clostridiaceae* strains may modulate signalling in the central, autonomic and enteric nervous systems; may modulate the activity of the HPA axis pathway; may modulate neuroendocrine and/or neuroimmune pathways; and may modulate the levels of commensal metabolites, inflammatory markers and/or gastrointestinal permeability of a subject, all of which are implicated in the neuropathology of bipolar disorder. In certain embodiments, treatment with *Clostridiaceae* strains may modulate the levels of oxytocin and/or vasopressin hormones. In preferred embodiments, the invention provides a composition comprising the strain deposited under accession number NCIMB 43454 for use in a method of treating or preventing bipolar disorder.

In further preferred embodiments, the invention provides a composition comprising a bacterial strain of the family *Clostridiaceae,* for use in a method of treating or preventing neurocognitive disorders, such as Alzheimer's disease. Compositions comprising a bacterial strain of the species family *Clostridiaceae* may improve cognitive and behavioural functioning in mouse models of neurocognitive disorders. Treatment with *Clostridiaceae* strains may modulate signalling in the central, autonomic and enteric nervous systems; may modulate the activity of the HPA axis pathway; may modulate neuroendocrine and/or neuroimmune pathways; and may modulate the levels of commensal metabolites and/or gastrointestinal permeability of a subject, all of which are implicated in the neuropathology of neurocognitive disorders. In preferred embodiments, the invention provides a composition comprising the strain deposited under accession number NCIMB 43454 for use in a method of treating or preventing neurocognitive disorders.

In further preferred embodiments, the invention provides a composition comprising a bacterial strain of the family *Clostridiaceae,* for use in a method of treating or preventing Parkinson's disease. Compositions comprising a bacterial strain of the family *Clostridiaceae* may improve motor and cognitive functions in mouse models of Parkinson's disease. Treatment with *Clostridiaceae* strains may modulate signalling in the central, autonomic and enteric nervous systems; may modulate the activity of the HPA axis pathway; may modulate neuroendocrine and/or neuroimmune pathways; and may modulate the levels of commensal metabolites, inflammatory markers and/or gastrointestinal permeability of a subject, all of which are implicated in the neuropathology of Parkinson's disease. In certain embodiments, treatment with *Clostridiaceae* strains may modulate the levels of oxytocin and/or vasopressin hormones. In preferred embodiments, the invention provides a composition comprising the strain deposited under accession number NCIMB 43454 for use in a method of treating or preventing Parkinson's disease.

In certain embodiments, the compositions of the invention are for use in a method of modulating the microbiota-gut-brain axis in the treatment or prevention of a disease or condition mediated by the microbiota-gut-brain axis. In particular, the compositions of the invention may be used in modulating the microbiota-gut-brain axis in the treatment or prevention of autism spectrum disorders; obsessive compulsive disorder; major depressive disorder; anxiety disorders; stress disorders; schizophrenia spectrum disorders; bipolar disorders; neurocognitive disorders and Parkinson's disease.

In preferred embodiments of the invention, the bacterial strain in the composition is the strain deposited under accession number NCIMB 43454. Closely related strains may also be used, such as bacterial strains that have a 16S rRNA sequence that is at least 95%, 96%, 97%, 98%, 99%, 99.5% or 99.9% identical to the 16S rRNA sequence of the strain deposited under accession number NCIMB 43454. Preferably, the bacterial strain has a 16S rRNA sequence that is at least 95%, 96%, 97%, 98%, 99%, 99.5% or 99.9% identical to SEQ ID NO: 1. Preferably, the bacterial strain for use in the invention has the 16S rRNA sequence represented by SEQ ID NO: 1. Bacterial strain which have at least 95%, 96%, 97%, 98%, 99%, 99.5% or 99.9% identity to the genome sequence of the bacterial strain deposited under accession number NCIMB 43454 may also be used. Most preferably, the bacterial strain in the composition is the strain deposited under accession number NCIMB 43454.

In certain embodiments, the composition of the invention is for oral administration. Oral administration of the strains of the invention can be effective for treating central nervous system disorders and conditions, in particular those mediated by the microbiota-gut-brain axis. Also, oral administration is convenient for patients and practitioners and allows delivery to and/or partial or total colonisation of the intestine.

In certain embodiments, the composition of the invention comprises one or more pharmaceutically acceptable excipients or carriers.

In certain embodiments, the composition of the invention comprises a bacterial strain that has been lyophilised. Lyophilisation is an effective and convenient technique for preparing stable compositions that allow delivery of bacteria.

In certain embodiments, the invention provides a food product comprising the composition as described above.

In certain embodiments, the invention provides a vaccine composition comprising the composition as described above.

Additionally, the invention provides a method of treating or preventing a disease or condition mediated by dysfunction of the microbiota-gut-brain axis, comprising administering a composition comprising a bacterial strain of the family *Clostridiaceae.*

In developing the above invention, the inventors have identified and characterised bacterial strains that are particularly useful for therapy. The strain deposited under accession number NCIMB 43454 of the invention has been shown to be particularly effective for treating the diseases described herein, such as autism spectrum disorder. Therefore, in another aspect, the invention provides a cell of the strain deposited under accession number NCIMB 43454, or a derivative thereof. The invention also provides a composition comprising such cells, or biologically pure cultures of such cells. The invention also provides a cell of the strain deposited under accession number NCIMB 43454, or a derivative thereof, for use in therapy, in particular for the diseases described herein.

In especially preferred embodiments, the invention provides a composition comprising the strain deposited under accession number NCIMB 43454, for use in a method of treating or preventing a central nervous system disorder or condition. In especially preferred embodiments, the invention provides a composition comprising the strain deposited under accession number NCIMB 43454, for use in a method of treating or preventing a neurodevelopmental disorder or a neuropsychiatric condition. In especially preferred embodiments, the invention provides a composition comprising the strain deposited under accession number NCIMB 43454, for use in a method of treating or preventing autism spectrum disorder, or preferably autism. In especially preferred embodiments, the invention provides a composition comprising the strain deposited under accession number NCIMB 43454, for use in a method of reducing stereotyped, repetitive, compulsive or anxious behaviour, especially in the treatment of autism.

In some embodiments, the bacterial strain may belong to the genus Intestinimonas instead of the family *Clostridiaceae.*

Further numbered embodiments of the invention are provided below:
1. A composition comprising a bacterial strain of the family *Clostridiaceae* for use in therapy.
2. A composition comprising a bacterial strain of the family *Clostridiaceae* for use in a method of treating or preventing a central nervous system disorder or condition.
3. The composition of embodiment 1 or 2, wherein the bacterial strain has at least 95%, 96%, 97%, 98%, 99%, 99.5% or 99.9% identity to the bacterial strain deposited under NCIMB 43454.
4. The composition of any preceding embodiment, wherein the bacterial strain has a 16S rRNA sequence that is at least 95%, 96%, 97%, 98%, 99%, 99.5% or 99.9% identical to SEQ ID NO: 1 or wherein the bacterial strain has the 16S rRNA sequence represented by SEQ ID NO: 1
5. A composition comprising a bacterial strain wherein the bacterial strain has a 16S rRNA sequence that is at least 95%, 96%, 97%, 98%, 99%, 99.5% or 99.9% identical to the 16S rRNA sequence of a bacterial strain of *Clostridiaceae,* for use in therapy.
6. A composition comprising a bacterial strain, wherein the bacterial strain has a 16S rRNA sequence that is at least 95%, 96%, 97%, 98%, 99%, 99.5% or 99.9% identical to SEQ ID NO: 1 or wherein the bacterial strain has the 16S rRNA sequence represented by SEQ ID NO: 1, for use in therapy.
7. A composition comprising a bacterial strain, wherein the bacterial strain has a 16S rRNA sequence that is least 95%, 96%, 97%, 98%, 99%, 99.5% or 99.9% identical to SEQ ID NO: 1 or wherein the bacterial strain has the 16S rRNA sequence represented by SEQ ID NO: 1, for use in a method of treating or preventing a central nervous system disorder or condition.
8. The composition of any one of embodiments 5-7, wherein the bacterial strain has a 16S rRNA sequence that is at least 99.5% or 99.9% identical to SEQ ID NO: 1.
9. The composition of any one of embodiments 2 and 6, wherein the central nervous system disorder or condition is mediated by the microbiota-gut-brain axis.
10. The composition of any one of embodiments 1-9, wherein the composition is for use in treating or preventing a neurodevelopmental disorder or a neuropsychiatric condition.
11. The composition of embodiment 10, wherein the composition is for use in a method of treating or preventing a disorder or condition selected from the group consisting of autism spectrum disorders (ASDs); child developmental disorder; obsessive compulsive disorder (OCD); major depressive disorder; depression; seasonal affective disorder; anxiety disorders; chronic fatigue syndrome (myalgic encephalomyelitis); stress disorder; post-traumatic stress disorder; schizophrenia spectrum disorders; schizophrenia; bipolar disorder; psychosis; mood disorder; dementia; Alzheimer's; Parkinson's disease; chronic pain; motor neuron disease; Huntington's disease; Guillain-Barré syndrome and meningitis.
12. The composition of embodiment 11 wherein the composition is for use in a method of treating or preventing autism spectrum disorder.
13. The composition of embodiment 12, wherein the composition is for use in a method of reducing or preventing autism.
14. The composition of any preceding embodiment, wherein the composition prevents, reduces or alleviates stereotyped, repetitive, compulsive or anxious behaviour.
15. The composition of any one of embodiments 1-9, wherein the composition is for use in a method of treating or preventing obsessive compulsive disorder.
16. The composition of embodiment 15, wherein the composition prevents, reduces or alleviates repetitive, compulsive and/or anxious behaviour.
17. The composition of any one of embodiments 1-9, wherein the composition is for use in in a method of treating or preventing major depressive disorder.
18. The composition of embodiment 17, wherein the composition treats or prevents acute major depressive episodes and/or the prevention of new episodes (recurrence prevention).
19. The composition of embodiment 17 or embodiment 18, wherein the composition prevents, reduces or alleviates the occurrence of mild, moderate or severe MDD episodes.
20. The composition of any ne of embodiments 1-9, wherein the composition is for use in in a method of treating or preventing anxiety disorders.
21. The composition of embodiment 18, wherein the anxiety disorder is generalised anxiety disorder (GAD); specific phobia; social anxiety disorder; separation anxiety disorder; agoraphobia; panic disorder and/or selective mutism.
22. The composition of any one of embodiments 1-9, wherein the composition is for use in a method of treating or preventing neurocognitive disorders.
23. The composition of embodiment 22, wherein the neurocognitive disorder is vascular dementias; mixed form Alzheimer's disease and vascular dementia; Lewy body disease; frontotemporal dementia; Parkinson's dementia; Creutzfeldt-Jakob disease; Huntington's disease; and Wernicke-Korsakoff syndrome.
24. The composition of any preceding embodiment, wherein the composition is for use in a method of modulating the microbiota-gut-brain axis.
25. The composition of any preceding embodiment, wherein the composition is for oral administration.
26. The composition of any preceding embodiment, wherein the composition comprises one or more pharmaceutically acceptable excipients or carriers.
27. The composition of any preceding embodiment, wherein the bacterial strain is lyophilised.
28. The composition of any preceding embodiment, wherein the bacterial strain is viable and capable of partially or totally colonising the intestine.
29. The composition of any preceding embodiment, wherein the composition comprises a single species of *Clostridiaceae.*
30. The composition of any preceding embodiment, wherein the composition comprises a single strain of *Clostridiaceae.*
31. The composition of any preceding embodiment, wherein the composition comprises the bacterial strain of *Clostridiaceae* as part of a microbial consortium.
32. The composition of claim 31, wherein the composition comprises fewer than 10, fewer than 9, fewer than 8, fewer than 7, fewer than 6, fewer than 5, fewer than 4, or fewer than 3 bacterial species.
33. A food product comprising the composition of any preceding embodiment, for the use of any preceding embodiment.
34. A vaccine composition comprising the composition of any preceding embodiment, for the use of any preceding embodiment.
35. A method of treating or preventing a central nervous system disorder or condition, comprising administering a composition comprising a bacterial strain of the family *Clostridiaceae* to a patient in need thereof.
36. The composition, food product, vaccine composition or method of any preceding embodiment, wherein the bacterial strain is not pathogenic.
37. The composition, food product, vaccine composition or method of any one of embodiments 1 to 35, wherein the bacterial strain is not *C. difficile.*
38. A cell of the *Clostridiaceae* strain deposited under accession number NCIMB 43454, or a derivative thereof, for the use of any of embodiments 1-32.
39. A composition comprising the cell of embodiment 38, for the use of embodiment 38.
40. The composition of embodiment 39, comprising a pharmaceutically acceptable carrier or excipient.
41. A biologically pure culture of *Clostridiaceae* strain deposited under accession number NCIMB 43454, or a derivative thereof.

### BRIEF DESCRIPTION OF DRAWINGS

**Figure 1** shows the effect on permeability of ileum (A) and colon (B) of strain NCIMB 43454, measured using fluorescence. *=p≤0.05.
**Figure 2** shows the effect of NCIMB 43454 on expression of TPH1 in the ileum, normalized to β-actin. *=p≤0.05.
**Figure 3** shows the effect of NCIMB 43454 on the concentration of the short-chain fatty acids in the caecal content.
**Figure 4** shows the effect of NCIMB 43454 on the expression CRFR1 (A) and Grin2B (B) in the hippocampus, BDNF (C), glucocorticoid receptor (D), mineralocorticoid receptor (E) and Grin2A (F) in the amygdala, and BDNF (G), TLR4 (H), CRFR1 (I), mineralocorticoid receptor (J) and GABA BR1 (K) in the prefrontal cortex (PFC), normalised to β-actin. *= p≤0.05.
**Figure 5** shows the results of elevated plus maze test in Btrb (A and B) and MIA (C and D) mice. Panels A and C show the number of entries into the open arm, and panels B and D show the total duration of these entries , expressed in s (B) or as a percentage of the total time spent in the maze (D).*= p≤0.05.
**Figure 6** shows the setup of the experiment to measure the time spent with social stimuli compared with non-social stimuli (A) and time spent exploring a novel animal compared with familiar animal (B). The duration is expressed in s. Panels C and D show the results for Btbr mice and panels E and F for MIA mice. **= p<0.01 and ****= p<0.0001.

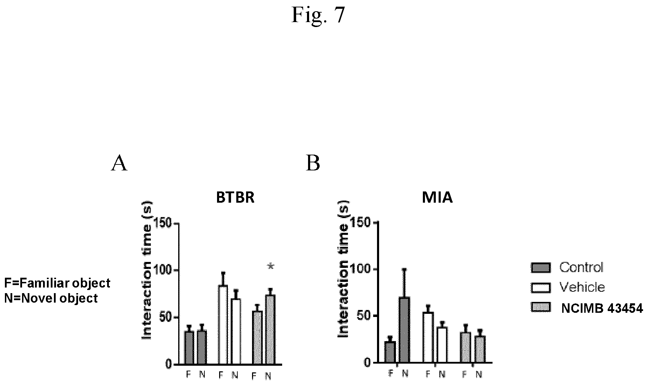

**Figure 7** shows the results of the novel object recognition test in Btbr (A) and MIA (B) mice. *= p≤0.05.
**Figure 8** shows the results of the forced swim test for Btbr (A) and MIA (B) mice. **= p≤0.01.
**Figure 9** shows the levels of noradrenaline (A), serotonin (B) and 5-HIAA/5-HT turnover (C) for Btbr mice and of the same monoamines in MIA mice (panels D-F, respectively). *= p≤0.05.
**Figure 10** shows expression of CRF2R (A), 5HT1AR (B), OXTR (C) and Grin2A (D) in the amygdala of MIA mice, normalized to β-actin. *= p≤0.05.

### DISCLOSURE OF THE INVENTION

### Bacterial strains

The compositions of the invention comprise a bacterial strain of the family *Clostridiaceae.* The examples demonstrate that bacteria of this family are useful in therapy, particularly for treating or preventing central nervous system disorders and conditions such as those mediated by the microbiota-gut-brain axis. The compositions of the invention are particularly useful for the treatment and prevention of autism spectrum disorders. The inventors have shown that the compositions of the invention can be used to treat such conditions, as shown in the examples. The mouse model experiments used in this application for the assessment of the symptoms of autism spectrum disorders are known in the art to be applicable for the assessment of the symptoms other central nervous system disorders including those listed herein.

In certain embodiments, the compositions of the invention comprise only one or more strains of the family *Clostridiaceae* and do not contain any other bacterial genera. In certain embodiments, the compositions of the invention comprise a single strain of the family *Clostridiaceae* and do not contain any other bacterial strains, genera or species. In certain embodiments, the compositions of the invention comprise a single species of the family *Clostridiaceae* and do not contain any other bacterial genera or species.

Examples of *Clostridiaceae* strains for use in the invention include the strain deposited under accession number NCIMB 43454. Bacteria of *Clostridiaceae* family have generally been described as Gram-positive, motile and obligate anaerobic bacteria that are rod-shaped, and which are capable of producing endospores [26].

The *Clostridiaceae* strain bacterium deposited under accession number NCIMB 43454 was tested in the Examples. A 16S rRNA sequence for this strain is provided in SEQ ID NO: 1. The strain was deposited with the international depositary authority NCIMB, Ltd. (Ferguson Building, Aberdeen, AB21 9YA, Scotland) by to 4D Pharma Research Limited (Life Sciences Innovation Building, Aberdeen, AB25 2ZS, Scotland) on 9th August 2019 as *"Clostridiacae* bacterium" and was assigned accession number NCIMB 43454.

In preferred embodiments the bacterial strain is not a pathogenic *Clostridiaceae* strain. In further preferred embodiments, the bacterial strain is not *C. difficile.*

Bacterial strains closely related to the strain tested in the examples are also expected to be effective for therapy, including for treating or preventing central nervous system disorders and conditions as described herein, in particular central nervous system disorders and conditions mediated by the microbiota-gut-brain axis. In certain embodiments, the bacterial strain for use in the invention has a 16S rRNA sequence that is at least 95%, 96%, 97%, 98%, 99%, 99.5% or 99.9% identical to the 16S rRNA sequence of a bacterial strain of *Clostridiaceae.* In certain embodiments, the bacterial strain for use in the invention has a 16S rRNA sequence that is at least 95%, 96%, 97%, 98%, 99%, 99.5% or 99.9% identical to the 16S rRNA sequence of the *Clostridiaceae* strain deposited under accession number NCIMB 43454. Preferably, the bacterial strain for use in the invention has a 16S rRNA sequence that is at least 95%, 96%, 97%, 98%, 99%, 99.5% or 99.9% identical to SEQ ID NO: 1. Preferably, the sequence identity is to SEQ ID NO: 1. Preferably, the bacterial strain for use in the invention has the 16S rRNA sequence represented by SEQ ID NO: 1.

Preferably, the bacterial strain has a 16s rRNA sequence that has at least 98.65% sequence similarity to SEQ ID NO: 1. Pairwise similarities between 16S rRNA gene sequences can be calculated based on robust global sequence alignment algorithms such as the EzTaxon server described in [27].

Bacterial strains that are biotypes of the bacterium deposited under accession number NCIMB 43454 are also expected to be effective for use in therapy, including for treating or preventing central nervous system disorders and conditions as described herein, in particular central nervous system disorders and conditions mediated by the microbiota-gut-brain axis. A biotype is a closely related strain that has the same or very similar physiological and biochemical characteristics. For example, a biotype strain will be able to increase acetate, butyrate and/or propionate production by at least about 5%, at least about 10%, at least about 20%, at least about 30%, at least about 40%, or at least about 50% compared to a control when administered to BALB/c mice, measured using a Varian 3500 GC flame-ionization system, fitted with a with a ZB-FFAP column. In addition, or alternatively, it may reduce depressive-like behaviour in Btbr or MIA mice, as measured by a decrease in immobility time in a forced swim test of at least about 5%, at least about 10%, at least about 20%, at least about 30%, at least about 40%, or at least about 50% compared to a vehicle-only control. In addition, or alternatively, it will be able to decrease permeability of the ileum of BALB/c mice by at least about 5%, at least about 10%, at least about 20%, at least about 30%, at least about 40%, or at least about 50% compared to a control, measured using Ussing chambers with oxygenated (95% O2, 5% CO2) Krebs buffer maintained at 37°C.

Strains that are biotypes of the bacterium deposited under accession number NCIMB 43454 and that are suitable for use in the invention may be identified by sequencing other nucleotide sequences for the bacterium deposited under accession number NCIMB 43454. For example, substantially the whole genome may be sequenced and a biotype strain for use in the invention may have at least 95%, 96%, 97%, 98%, 99%, 99.5% or 99.9% sequence identity across at least 80% of its whole genome (e.g. across at least 85%, 90%, 95% or 99%, or across its whole genome). For example, in some embodiments, a biotype strain has at least 98% sequence identity across at least 98% of its genome or at least 99% sequence identity across 99% of its genome. Other suitable sequences for use in identifying biotype strains may include hsp60 or repetitive sequences such as BOX, ERIC, (GTG)s, or REP or [28]. Biotype strains may have sequences with at least 95%, 96%, 97%, 98%, 99%, 99.5% or 99.9% sequence identity to the corresponding sequence of the bacterium deposited under accession number NCIMB 43454.

In some embodiments, a biotype strain comprises a 16S rRNA sequence with at least 95%, 96%, 97%, 98%, 99%, 99.5% or 99.9% sequence identity to SEQ ID NO: 1. In some embodiments, a biotype strain has the 16S rRNA sequence of SEQ ID NO: 1.

Alternatively, strains that are biotypes of a bacterium deposited under accession number NCIMB 43454 and that are suitable for use in the invention may be identified by using the accession number NCIMB 43454 deposit, and restriction fragment analysis and/or PCR analysis, for example by using fluorescent amplified fragment length polymorphism (FAFLP) and repetitive DNA element (rep)-PCR fingerprinting, or protein profiling, or partial 16S or 23S rDNA sequencing. In preferred embodiments, such techniques may be used to identify other *Clostridiaceae* strains.

In certain embodiments, strains that are biotypes of a bacterium deposited under accession number NCIMB 43454 and that are suitable for use in the invention are strains that provide the same pattern as a bacterium deposited under accession number NCIMB 43454 when analysed by amplified ribosomal DNA restriction analysis (ARDRA), for example when using Sau3AI restriction enzyme (for exemplary methods and guidance see, for example, [29]). Alternatively, biotype strains are identified as strains that have the same carbohydrate fermentation patterns as a bacterium deposited under accession number NCIMB 43454.

Other *Clostridiaceae* strains that are useful in the compositions and methods of the invention, such as biotypes of the bacterium deposited under accession number NCIMB 43454, may be identified using any appropriate method or strategy, including the assays described in the examples. For instance, strains for use in the invention may be identified by culturing in anaerobic YCFA and/or administering the bacteria to an autism spectrum disorder mouse model and then assessing cytokine levels. In particular, bacterial strains that have similar growth patterns, metabolic type and/or surface antigens to the bacterium deposited under accession number NCIMB 43454 may be useful in the invention. In particular, a biotype strain will elicit comparable effects on the autism spectrum disorder models to the effects shown in the Examples, for example, in social behaviour and forced swim tests.

A particularly preferred strain of the invention is the *Clostridiaceae* strain deposited under accession number NCIMB 43454. This is an exemplary *Clostridiaceae* strain tested in the examples and shown to be effective for treating disease. Therefore, the invention provides a cell, such as an isolated cell, of the *Clostridiaceae* strain deposited under accession number NCIMB 43454, or a derivative thereof. The invention also provides a composition comprising a cell of the *Clostridiaceae* strain deposited under accession number NCIMB 43454, or a derivative thereof. The invention also provides a biologically pure culture of the *Clostridiaceae* strain deposited under accession number NCIMB 43454. The invention also provides a cell of the *Clostridiaceae* strain deposited under accession number NCIMB 43454, or a derivative thereof, for use in therapy, in particular for the diseases described herein.

A derivative of the strain deposited under accession number NCIMB 43454 may be a daughter strain (progeny) or a strain cultured (subcloned) from the original. A derivative of a strain of the invention may be modified, for example at the genetic level, without ablating the biological activity. In particular, a derivative strain of the invention is therapeutically active. A derivative strain will have comparable immune modulatory activity to the original strain. In particular, a derivative strain will elicit comparable effects on the central nervous system disorder or condition models and comparable effects on cytokine levels to the effects shown in the Examples, which may be identified by using the culturing and administration protocols described in the Examples. A derivative of the NCIMB 43454 strain will generally be a biotype of the NCIMB 43454 strain.

The bacterial strain may also be a strain that has the same safety and therapeutic efficacy characteristics as the strain deposited under accession number NCIMB 43454, and such cells are encompassed by the invention.

In preferred embodiments, the bacterial strains in the compositions of the invention are viable and capable of partially or totally colonising the intestine.

### Therapeutic uses

The bacterial strains described herein are useful for therapy, in particular for treating and preventing a central nervous system disorder or condition . In general, a therapeutic effect does not necessarily mean a complete cure of the disease necessarily. Rather, a therapeutic effect may also be given where one or more symptoms of the disease are ameliorated or cured.

### Modulation of the microbiota-gut-brain axis

Communication between the gut and the brain (the microbiota-gut-brain axis) occurs via a bidirectional neurohumoral communication system. Recent evidence shows that the microbiota that reside in the gut can modulate brain development and produce behavioural phenotypes via the microbiota-gut-brain axis. Indeed, a number of reviews suggest a role of the microbiota-gut-brain axis in maintaining central nervous system functionality and implicate dysfunction of the microbiota-gut-brain axis in the development of central nervous system disorders and conditions [10],[13],[14],[30].

The bidirectional communication between the brain and the gut (*i.e.* the-gut-brain axis) includes the central nervous system, neuroendocrine and neuroimmune systems, including the hypothalamus-pituitary-adrenal (HPA) axis, sympathetic and parasympathetic arms of the autonomic nervous system (ANS), including the enteric nervous system (ENS) and the vagus nerve, and the gut microbiota.

As demonstrated in the examples, the compositions of the invention can modulate the microbiota-gut-brain axis and reduce behavioural symptoms associated with a CNS disorder. Accordingly, the compositions of the invention may be useful for treating or preventing disorders of the central nervous system (CNS), in particular those disorders and conditions associated with dysfunction of the microbiota-gut-brain axis.

The compositions of the invention may also be useful for treating or preventing neurodevelopmental disorders and/or neuropsychiatric conditions. Neurodevelopmental diseases and neuropsychiatric conditions are often associated with the microbiota-gut-brain axis. The compositions of the invention may be useful for treating or preventing neurodevelopmental diseases and/or neuropsychiatric conditions mediated by dysfunction of the microbiota-gut-brain axis. In further preferred embodiments, the compositions of the invention are for use in treating or preventing a neurodevelopmental disorder or a neuropsychiatric condition.

In particular embodiments, the compositions of the invention may be useful for treating or preventing a disease or condition selected from the group consisting of: autism spectrum disorders (ASDs); child developmental disorder; obsessive compulsive disorder (OCD); major depressive disorder; depression; seasonal affective disorder; anxiety disorders; schizophrenia spectrum disorders; schizophrenia; bipolar disorder; psychosis; mood disorder; chronic fatigue syndrome (myalgic encephalomyelitis); stress disorder; post-traumatic stress disorder; dementia; Alzheimer's; Parkinson's disease; and/or chronic pain. In further embodiments, the compositions of the invention may be useful for treating or preventing motor neuron disease; Huntington's disease; Guillain-Barre syndrome and/or meningitis.

The compositions of the invention may be particularly useful for treating or preventing chronic disease, treating or preventing disease in patients that have not responded to other therapies (such as treatment with anti-psychotics and/or anti-depressants), and/or treating or preventing the tissue damage and symptoms associated with dysfunction of the microbiota-gut-brain axis.

In certain embodiments, the compositions of the invention modulate the CNS. In some embodiments, the compositions of the invention modulate the autonomic nervous system (ANS). In some embodiments, the compositions of the invention modulate the enteric nervous system (ENS). In some embodiments, the compositions of the invention modulate the hypothalamic, pituitary, adrenal (HPA) axis. In some embodiments, the compositions of the invention modulate the neuroendocrine pathway. In some embodiments, the compositions of the invention modulate the neuroimmune pathway. In some embodiments, the compositions of the invention modulate the CNS, the ANS, the ENS, the HPA axis and/or the neuroendocrine and neuroimmune pathways. In certain embodiments, the compositions of the invention module the levels of commensal metabolites and/or the gastrointestinal permeability of a subject.

The signalling of the microbiota-gut-brain axis is modulated by neural systems. Accordingly, in some embodiments, the compositions of the invention modulate signalling in neural systems. In certain embodiments, the compositions of the invention modulate the signalling of the central nervous system. In some embodiments, the compositions of the invention modulate signalling in sensory neurons. In other embodiments, the compositions of the invention modulate signalling in motor neurons. In some embodiments, the compositions of the invention modulate the signalling in the ANS. In some embodiments, the ANS is the parasympathetic nervous system. In preferred embodiments, the compositions of the invention modulate the signalling of the vagus nerve. In other embodiments, the ANS is the sympathetic nervous system. In other embodiments, the compositions of the invention modulate the signalling in the enteric nervous system. In certain embodiments, the signalling of ANS and ENS neurons responds directly to luminal contents of the gastrointestinal tract. In other embodiments, the signalling of ANS and ENS neurons responds indirectly to neurochemicals produced by luminal bacteria. In other embodiments, the signalling of ANS and ENS neurons responds to neurochemicals produced by luminal bacteria or enteroendocrine cells. In certain preferred embodiments, the neurons of the ENS activate vagal afferents that influence the functions of the CNS. In some embodiments, the compositions of the invention regulate the activity of enterochromaffin cells.

In certain embodiments, the compositions of the invention modulate fear conditioning in an animal model. In certain embodiments, the compositions of the invention can be used to modulate the development of fear and/or anxiety, and/or modulate the extent to which the fear and/or anxiety becomes extinct in a subject. In certain embodiments, the compositions of the invention can be used to modulate the extent of stress-induced hyperthermia in an animal model. In certain embodiments, the compositions of the invention modulate the level of stress and/or anxiety in a subject.

### Autism spectrum disorder (ASD)

Autism spectrum disorder is a set of heterogeneous neurodevelopmental conditions, characterised by early-onset difficulties in social interaction, communication and unusually restricted, repetitive behaviour and interests. Symptoms can be recognised from a very early age but ASD is often diagnosed in more able children starting mainstream education. Autism represents the primary type of ASD.

Historically, autism has been diagnosed on the basis of three core domains: impaired social interaction, abnormal communication, and restricted and repetitive behaviours and interests. In the International Classification of Diseases (ICD-10R, WHO 1993) and the Diagnostic and Statistical Manual (DSM-IV, American Psychiatric Association, 2000), autism comes under the umbrella term of Pervasive Developmental Disorder (PDD), with four possible diagnostic subtypes: Asperger Syndrome, Childhood Autism/Autistic Disorder, Atyptical Autism, and PDD-not otherwise specified. In DMS-5, these diagnostic subtypes are combined into a single category of autism spectrum disorder (ASD) and the previous use of three core domains of impairment has been reduced to two main areas, namely social communication and interaction, and repetitive behaviour, which include sensory integration dysfunctions.

ASD is a 'spectrum disorder' as it affects each person in a variety of different ways and can range from very mild to severe. The functioning of the affected individual varies substantially depending on language abilities, level of intelligence, co-morbidity, composition of symptoms and access to services. Cognitive functioning, learning, attention and sensory processing are usually impaired.

DSM-IV states that the diagnosis of autism requires the presence of at least six symptoms, including a minimum of two measures of qualitative impairment in social interaction, one symptom of qualitative impairment in communication, and one symptom of restricted and repetitive behaviour. DMS-5 redefines diagnosis of ASD into two symptom domains: (i) social interaction and social communication deficits; and (ii) restricted, repetitive patterns of behaviour, interests or activities.

Co-morbid medical conditions are highly prevalent in ASDs. Co-morbid include anxiety and depression, seizures, attention deficits, aggressive behaviours, sleep problems, gastrointestinal disorders, epilepsy, mental retardation, intellectual disabilities and feeding difficulties.

The examples demonstrate that the compositions of the invention achieve a reduction in disease incidence and disease severity in an animal model of autism spectrum disorder and so they may be useful in the treatment or prevention of autism spectrum disorders.

ASD is a central nervous system disorder that is partially triggered by environmental factors. Therefore, dysfunction of the microbiota-gut-brain axis may be responsible for development and persistence of ASDs. Accordingly, in preferred embodiments, the composition of the invention are for use in treating or preventing autism spectrum disorders. In some embodiments, the compositions of the invention are for use in treating or preventing autism. In some embodiments, the autism is Pervasive Developmental Disorder (PDD). In another embodiment, the PDD is Asperger Syndrome, Childhood Autism/Autistic Disorder, Atyptical Autism and/or PDD-not otherwise specified. Accordingly, in some embodiments, the compositions of the invention are for use in treating or preventing autism spectrum disorders, autism, pervasive developmental disorder; Asperger Syndrome; Childhood Autism/Autistic Disorder, Atypical Autism and/or PDD-not otherwise specified.

The compositions of the invention may be useful for modulating the microbiota-gut-brain axis of a subject. Accordingly, in preferred embodiments the compositions of the invention are for use in preventing an ASD in a patient that has been identified as at risk of an ASD, or that has been diagnosed with an ASD at a prenatal or an early developmental stage; in childhood and/or in adulthood. The compositions of the invention may be useful for preventing the development of ASDs.

The compositions of the invention may be useful for managing or alleviating ASDs. Treatment or prevention of ASDs may refer to, for example, an alleviation of the severity of symptoms or a reduction in the frequency of exacerbations or the range of triggers that are a problem for the patient.

In preferred embodiments, the compositions of the invention prevent, reduce or alleviate at least one symptom of ASDs.

In some embodiments, the compositions of the invention prevent, reduce or alleviate at least one of the two symptom domains of ASD classified in the DMS-5. In some embodiments, the compositions of the invention prevent, reduce or alleviate social interaction and/or social communication deficits. In some embodiments, the compositions of the invention prevent, reduce or alleviate restrictive, repetitive patterns of behaviour, interests or activities. In some embodiments, the compositions of the invention prevent, reduce or alleviate social interaction, social communication deficits and/or restrictive, repetitive patterns of behaviour, interests or activities.

In some embodiments, the compositions of the invention prevent, reduce or alleviate repetitive behaviour, stereotyped behaviour, compulsive behaviour, routine behaviour, sameness behaviour and restricted behaviour. In some embodiments, the compositions of the invention improve social awareness, social information processing, capacity for social communication, social anxiety/avoidance, and autistic preoccupations and traits in a subject with ASDs.

In some embodiments, the compositions of the invention prevent, reduce or alleviate additional symptoms associated with the core symptoms of ASDs. In some embodiments, the compositions of the invention prevent, reduce or alleviate irritability (including aggression, deliberate self-injury and temper tantrums), agitation, crying, lethargy, social withdrawal, stereotypic behaviour, hyperactivity, non-compliance, inappropriate speech, anxiety, depression, and/or over or under-controlled behaviour in a subject with ASDs. In some embodiments, the compositions of the invention improve cognitive functioning, learning, attention and/or sensory processing in a subject with ASD.

In other embodiments, the compositions of the invention improve secondary outcome measures in a subject with ASDs. In some embodiments, the secondary outcome measures include additional symptom and/or functional rating scales, behavioural scales and miscellaneous measures of interest.

In some embodiments, the compositions of the invention cause a positive change in the diagnostic and/or symptomatic scale for the assessment of core symptoms of a subject with ASDs. In some embodiments, the diagnostic and/or symptomatic scale is the Autism Diagnostic Interview - Revised (ASI-R). In some embodiments, the diagnostic or symptomatic scale is the Autism Diagnostic Observation Schedule-Generic (ADOS-G) now ADOS-2. In other embodiments, the diagnostic or symptomatic scale is the Autism Diagnostic Interview Revised (ADI-R). In other embodiments, the diagnostic or symptomatic scale is the Diagnostic Interview for Social and Communication Disorders (DISCO). In yet other embodiments, the diagnostic or symptomatic scale is the Childhood Autism Rating Scale (CARS and CARS2).

In some embodiments, the compositions of the invention cause a positive change in generic measures of the efficacy endpoints of ASDs. In certain embodiments, the generic measures include, but are not limited to the Aberrant Behaviour Checklist (ABC), the Child Behaviour Checklist (CBCL), the Vineland-II Adaptive Behaviour Scales (VABS), the Social Responsiveness Scale (SRS), and/or the Repetitive Behaviour Scale - Revised (RBS-R).

In some embodiments, the compositions of the invention improve the Clinical Global Impression - Global Improvement (CGI-I) scale for assessing psychiatric and neurological disorders. In some embodiments, the compositions of the invention display a positive effect on global functioning of the subject with ASDs.

Additional scales would be known to a person skilled in the art. In some embodiments, the compositions of the invention would improve the outcome of diagnostic and/or symptomatic scales known to a person skilled in the art.

In certain embodiments, the compositions of the invention prevent, reduce or alleviate the incidence of comorbidities of ASDs. In some embodiments, the compositions of the invention prevent, reduce or alleviate the incidence of anxiety and depression, seizures, attention deficits, aggressive behaviours, sleep problems, gastrointestinal disorders (including irritable bowel syndrome (IBS)), epilepsy, mental retardation, intellectual disabilities and/or feeding difficulties. In certain embodiments, the compositions of the invention prevent, reduce or alleviate gastrointestinal comorbidities, such as abdominal pain, diarrhoea and flatulence.

In some embodiments, the compositions of the invention prevent, reduce or alleviate the symptoms of certain psychiatric and behavioural disorders that may present clinically with similarities to autism. Accordingly, in some embodiments, the compositions of the invention, prevent, reduce or alleviate attention deficit disorder (ADHD); affective/anxiety disorders; attachment disorders; oppositional defiant disorder (ODD); obsessive compulsive disorder (OCD) and/or psychoses including schizophrenia (cognitive impairment).

In some embodiments, the compositions of the invention are particularly effective at preventing, reducing or alleviating ASDs when used in combination with another therapy for treating ASDs. Such therapies include anti-psychotic, anti-anxiety and anti-depressant drugs. Such drugs include risperidone (Risperdal®); olanzapine (Zyprexa®); fluoxetine (Prozac®); sertraline (Zoloft®); fluvoxamine (Luvox®); clomipramine (Anafranil®); haloperidol (Haldol®); thioridazine; fluphenazine; chlorpromazine; ziprasidone (Geogon®); carbamazepine (Tegretol®); lamotrigine (Lamictal®); topiramate (Topomax®); valproic acid (Depakote®); methylphenidate (Ritalin®); diazepam (Valium®) and lorazepam (Ativan®).

The EMA Guidelines on the clinical development of medicinal products for the treatment of autism spectrum disorder state that, due to the heterogeneity of the diseases, it may not be possible to achieve a significant effect on all core symptoms with a single compound, and so short term efficacy has to be demonstrated on at least one core symptom. The live biotherapeutic strains used in the Examples have shown effective treatment of at least one core symptom of autistic spectrum disorder, so these strains and related *Clostridiaceae* strains are expected to be effective against human disease.

### Obsessive compulsive disorder (OCD)

OCD is a heterogeneous, chronic and disabling disorder belonging to the anxiety disorders. According to the DSM-IV definition, the essential features of OCD are recurrent obsessions and/or compulsions (criterion A) that are severe and time consuming (more than one hour a day) or cause marked distress or significantly interfere with the subject's normal routine, occupational functioning, usual social activities or relationships (criterion C). As some point during the course of the disorder, the person has recognised that the obsessions or compulsions are excessive or unreasonable (criterion B).

Obsessions are defined as recurrent and persistent thoughts, impulses or images that are experienced as intrusive and inappropriate and cause marked anxiety or distress. The thoughts, impulses or images are not simply excessive worries about real-life problems, they are recognised by the patient as a product of his own mind (e.g. fear for contamination, symmetry obsession). The person attempts to ignore, suppress or neutralise the obsessions with some other thoughts or actions.

Compulsions are defined as repetitive behaviours (e.g. hand washing, ordering, hoarding, checking) or mental acts (e.g. praying, counting, repeating words silently) that the person feels driven to perform in response to an obsession or according to rules that must be applied rigidly.

OCD is often associated with co-morbidity rates of other psychiatric diseases including major depressive disorder, other anxiety disorders (generalised anxiety disorder, social anxiety disorder, panic disorder), substance abuse and eating disorders (anorexia and bulimia).

OCD is a psychiatric disorder that may develop or persist due to dysfunction of the microbiota-gut-brain axis. Accordingly, in preferred embodiments, the compositions of the invention are for use in treating or preventing OCD in a subject.

In certain embodiments, the compositions of the invention prevent, reduce or alleviate the essential symptomatic features of OCD. In certain embodiments, the compositions of the invention prevent, reduce or alleviate recurrent obsessions and/or compulsions in a subject. In certain embodiments, the obsessions are recurrent or persistent thoughts, impulses or images that are experiences as intrusive and inappropriate and cause marked anxiety or distress. In certain embodiments, the compulsions are repetitive behaviours that the subject feels driven to perform in response to an obsession or according to rules that must be applied rigidly.

In certain embodiments, the compositions of the invention improve symptoms of OCD in a subject accordingly to the Y-BOCS and/or the NIMH-OC diagnostic and/or symptomatic scales. In some embodiments, the Y-BOCS scale is used to monitor improvement of primary endpoints. In some embodiments, the NIMH-OC scale is used to monitor improvement of secondary parameters.

In some embodiments, the compositions of the invention improve the Clinical Global Impression - Global Improvement (CGI-I) scale for assessing psychiatric and neurological disorders. In some embodiments, the compositions of the invention display a positive effect on global social functioning (relationships, work, *etc*.) of the subject with ASDs. In some embodiments, the global scale is the Sheehan disability scale.

In preferred embodiments, the compositions of the invention prevent, reduce or alleviate at least one comorbidity of OCD. The comorbidities of OCD include major depressive disorder, other anxiety disorders (generalised anxiety disorder, social anxiety disorder, panic disorder), substance abuse and eating disorders (anorexia and bulimia) Gilles de la Tourette syndrome, ADHD (Attention-Deficit/Hyperactivity Disorder) and developmental disorders.

In some embodiments, the compositions of the invention are particularly effective at preventing, reducing or alleviating OCD when used in combination with another therapy for treating OCD. Such therapies include serotonin and dopamine reuptake inhibitors; clomipramine and anti-psychotics.

### Major depressive disorder (MDD)

MDD is associated with substantial psychosocial dysfunction and high individual mental strain as well as with excess morbidity and mortality (the risk of suicide is considerable). The term major depressive disorder encompasses clinical depression, major depression, unipolar depression, unipolar disorder, recurrent depression and simply depression. The term major depressive disorder covers mood disorders; dysthymia; chronic depression; seasonal affective disorder and borderline personality disorder.

According to the DMS-5 criteria, MDD symptoms include a depressed mood, or loss of interest or pleasure in daily activities for more than two weeks; and impaired social, occupational and educational function. Specific symptoms, at least five of the following nine, present nearly every day: depressed mood or irritable most of the day; decreased interest or pleasure in most activities, most of each day; significant weight change or change in appetite; change in sleep (insomnia or hypersomnia); change in activity (psychomotor agitation or retardation); fatigue or loss of energy; guilt or worthlessness (feelings of worthlessness or excessive or inappropriate guilt); reduced concentration (diminished ability to think or concentrate, or more indecisiveness; and suicidality (thoughts of death or suicide, or subject has a suicide plan). In addition, MDD is associated with anxiety symptoms including irrational worry; preoccupation with unpleasant worries; trouble relaxing and/or feeling tense. MDD episodes can be mild, moderate or severe.

MDD episodes are often associated with comorbidity with other psychiatric disorders or with somatic disorders like Parkinson's disease, Alzheimer's disease, cerebrovascular disorders, cancer and chronic pain syndromes. MDD is frequently associated with a wide spectrum of other mental disorders as comorbidities including generalised anxiety disorder; anxiety disorder; substance use disorders; post-traumatic stress disorder (PTSD); personality disorders; pain; stress; irritable bowel syndrome; insomnia; headaches and interpersonal problems.

Major depressive disorder is a psychiatric disorder that may develop or persist due to dysfunction of the microbiota-gut-brain axis. Accordingly, in preferred embodiments, the compositions of the invention are for use in treating or preventing MDD in a subject.

In certain embodiments, the compositions of the invention are for use in treating or preventing acute major depressive episodes and/or the prevention of new episodes (recurrence prevention). In certain embodiments, the compositions of the invention prevent, reduce or alleviate the occurrence of mild, moderate or severe MDD episodes.

In certain embodiments, the compositions of the invention prevent, reduce or alleviate one or more of the symptoms of MDD as classified by the DMS-5 criteria listed herein. In a preferred embodiment, the compositions of the invention prevent, reduce or alleviate a depressed mood in a subject. In a preferred embodiment, the compositions of the invention prevent, reduce or alleviate a decreased interest or pleasure in most activities in a subject. In some embodiments, the compositions of the invention reduce the occurrence of symptoms of MDD within a 2-week period.

In some embodiments, the compositions of the invention improve the symptoms of MDD according to a symptomatic or diagnostic scale. Such scales for assessing symptomatic improvement include the Hamilton Rating Scale of Depression (HAMD) and the Montgomery Asberg Depression Rating Scale. In addition, the Zung Self-Rating Depression Scale (SDS) and Zung Self-Rating Anxiety Scale (SAS) are also suitable symptomatic improvement scales.

In some embodiments, the compositions of the invention improve the Clinical Global Impression - Global Improvement (CGI-I) scale for assessing psychiatric and neurological disorders. In some embodiments, the compositions of the invention display a positive effect on global social and occupational functioning of the subject with MDD.

In certain embodiments, the compositions of the invention are for use in treating or preventing treatment resistant MDD.

In preferred embodiments, the compositions of the invention prevent, reduce or alleviate at least one comorbidity of MDD. The comorbidities of MDD include generalised anxiety disorder; anxiety disorder; substance use disorders; post-traumatic stress disorder (PTSD); personality disorders; pain; stress; IBS; insomnia; headaches and interpersonal problems.

In some embodiments, the compositions of the invention are particularly effective at preventing, reducing or alleviating MDD when used in combination with another therapy for treating MDD. Such therapies include antidepressants, augmentation strategies (e.g. combination therapy, lithium and other mood stabilizers, thyroid hormones and atypical antipsychotics) or even second generation antipsychotics.

### Anxiety disorders

Anxiety disorders are a group of mental disorders characterised by feelings of anxiety and fear. There are a number of anxiety disorders including generalised anxiety disorder (GAD); specific phobia; social anxiety disorder; separation anxiety disorder; agroraphobia; panic disorder and selective mutism.

GAD is diagnosed according to DMS-5 in six criterion. The first criterion is too much anxiety or worry over more than six months wherein the anxiety or worry is present most of the time in regards to many activities. The second criterion is that the subject is unable to manage the symptoms of the first criterion. The third criterion is that at least three (one in children) of the following occurs: restlessness; tires easily; problems concentrating; irritability; muscle tension and problems with sleep. The final three criterion are that the symptoms results in significant social, occupational and functional impairment; the symptoms are not due to medications, drugs, or other physical health problems; and the symptoms do not fit better with another psychiatric problem such as panic disorder. All other anxiety disorders may be considered as differential diagnoses of GAD.

GAD is frequently associated with a wide spectrum of other mental disorders as comorbidities including depression; substance use disorders; stress; IBS; insomnia; headaches; pain; cardiac events; interpersonal problems and ADHD.

Anxiety disorders are psychiatric disorders that may develop or persist due to dysfunction of the microbiota-gut-brain axis. Accordingly, in preferred embodiments, the compositions of the invention are for use in treating or preventing anxiety disorders in a subject. In certain embodiments, the anxiety disorder is generalised anxiety disorder (GAD); specific phobia; social anxiety disorder; separation anxiety disorder; agoraphobia; panic disorder and selective mutism.

In certain embodiments, the compositions of the invention prevent, reduce or alleviate one or more of the symptoms of GAD in a subject as classified by the DMS-5 criteria listed herein. According to DMS-5, the same symptoms are associated with other anxiety disorders. Therefore, in certain embodiments, the compositions of the invention prevent, reduce or alleviate one or more of the symptoms of anxiety disorders in a subject. In preferred embodiments, the compositions of the invention prevent, reduce or alleviate the anxiety or worry of the subject. In certain embodiments, the compositions of the invention reduce the occurrence of symptoms within a six month period. In certain embodiments, the composition of the invention prevents, reduces or alleviates restlessness; fatigue; loss of concentration; irritability; muscle tension; and/or problems with sleep. In some embodiments, the compositions of the invention prevent, reduce or alleviate social, occupational and functional impairment associated with anxiety disorders.

In some embodiments, the compositions of the invention improve the symptoms of anxiety disorders according to a symptomatic or diagnostic scale. In certain embodiments, the scale for assessing symptomatic improvement includes the Hamilton Anxiety Rating Scale (HAM-A). In some embodiments, the HAM-A total scale is used to assess primary endpoint. In other embodiments, the HAM-A psychic anxiety factor may be useful as a secondary endpoint.

In some embodiments, the compositions of the invention improve the Clinical Global Impression - Global Improvement (CGI-I) scale for assessing psychiatric and neurological disorders. In some embodiments, the compositions of the invention display a positive effect on global social, occupational and functional impairment of the subject with anxiety disorder. In some embodiments, the global scale is the Sheehan disability scale.

In preferred embodiments, the compositions of the invention prevent, reduce or alleviate at least one comorbidity of GAD and anxiety disorders. The comorbidities of GAD include depression; substance use disorders; stress; IBS; insomnia; headaches; pain; cardiac events; interpersonal problems and ADHD.

In some embodiments, the compositions of the invention are particularly effective at preventing, reducing or alleviating anxiety disorders when used in combination with another therapy for treating anxiety disorders. Such therapies include selective serotonin reuptake inhibitors (venlafaxine, duloxetine, escitalopram and paroxetine); benzodiazepines (alprazolam, lorazepam and clonazepam); pregabalin (Lyrica®) and gabapentin (Neurontin ®); serotonin receptor partial agonists (buspirone and tandospirone); atypical serotonergic antidepressants (such as imipramine and clomipramine); monoamine oxidase inhibitors (MAOIs) (such as moclobemide and phenelzine); hydroxyzine; propranolol; clonidine; guanfacine and prazosin.

### Post-traumatic stress disorder (PTSD)

PTSD is a severe and disabling disorder, an essential feature of which is the inclusion of a traumatic event as a precipitating factor of this disorder.

The symptoms of PTSD are grouped into four main clusters according to the DMS-V criteria: (i) intrusion: examples include nightmares, unwanted thoughts of the traumatic events, flashbacks, and reacting to traumatic reminders with emotional distress or physiological reactivity; (ii) avoidance: examples include avoiding triggers for traumatic memories including places, conversations, or other reminders; (iii) negative alterations in cognitions and mood: examples include distorted blame of self or others for the traumatic event, negative beliefs about oneself or the world, persistent negative emotions (e.g., fear, guilt, shame), feeling alienated, and constricted affect (e.g., inability to experience positive emotions); (iv) alterations in arousal and reactivity: examples include angry, reckless, or self-destructive behaviour, sleep problems, concentration problems, increased startle response, and hypervigilance.

Symptoms that resolve within 4 weeks of the traumatic event meet the criteria for an Acute Stress Disorder. The DSM distinguishes between acute (duration of symptoms for less than three months) and chronic PTSD (duration of symptoms longer than 3 months). If the symptoms begin more than 6 months after the stressor, the disorder is defined as delayed onset PTSD.

PTSD carries high comorbidities with major depressive disorder and substance use disorders.

PTSD is a psychiatric disorder that may develop or persist due to dysfunction of the microbiota-gut-brain axis. Accordingly, in preferred embodiments, the compositions of the invention are for use in treating or preventing PTSD in a subject. According to a similar pathogenesis, in certain embodiments, the compositions of the invention are for use in treating or preventing stress disorders. In certain embodiments, the compositions of the invention treat acute stress disorder. In some embodiments, the compositions of the invention treat acute and/or chronic PTSD. In some embodiments, the compositions of the invention treat delayed onset PTSD.

In certain embodiments, the compositions of the invention prevent, reduce or alleviate one or more of the symptoms of PTSD (or stress disorder) in a subject as classified by the DMS-5 criteria listed herein. In preferred embodiments, the compositions of the invention prevent, reduce or alleviate intrusive thoughts in a subject with PTSD. In preferred embodiments, the compositions of the invention prevent, reduce or alleviate avoidance behaviour in a subject with PTSD. In preferred embodiments, the compositions of the invention prevent, reduce or alleviate negative alterations in cognitions and mood in a subject with PTSD. In preferred embodiments, the compositions of the invention prevent alterations in arousal and reactivity in a subject with PTSD.

In some embodiments, the compositions of the invention improve the symptoms of PTSD and stress disorders according to a symptomatic or diagnostic scale. In certain embodiments, the scale for assessing symptomatic improvement is the Clinical-Administered PTSD (CAPS) scale.

In some embodiments, the compositions of the invention improve the Clinical Global Impression - Global Improvement (CGI-I) scale for assessing psychiatric and neurological disorders. In some embodiments, the compositions of the invention display a positive effect on global social, occupational and functional impairment of the subject with PTSD and stress disorders. In some embodiments, the global scale is the Sheehan disability scale.

In preferred embodiments, the compositions of the invention prevent, reduce or alleviate at least one comorbidity of PTSD and stress disorders. The comorbidities of PTSD and stress disorders include MDD, substance use disorders; stress and anxiety.

In some embodiments, the compositions of the invention are particularly effective at preventing, reducing or alleviating PTSD and stress disorders when used in combination with another therapy for treating PTSD and stress disorders. Such therapies include serotoninergic agents, tricyclic antidepressants, mood stabilisers, adrenergic inhibiting agents, antipsychotics, benzodiazepines, sertraline (Zoloft®), fluoxetine (Prozac®) and/or paroxetine (Paxil®).

### Schizophrenia spectrum and psychotic disorders

These diseases affect a subject's ability to think clearly, make good judgements, respond emotionally, communicate effectively, understand reality, and behave appropriately. Psychotic diseases include schizophrenia (symptoms listed below); schizoaffective disorder (the subject has symptoms of both schizophrenia and a mood disorder, such as depression or bipolar disorder); schizophreniform disorder (displays the symptoms of schizophrenia, but the symptoms last for a shorter time: between 1 and 6 months); brief psychotic disorder (subjects display a sudden, short period of psychotic behaviour, often in response to a very stressful event, such as a death in the family - recovery is usually less than a month); delusional disorder (delusions last for at least 1 month); shared psychotic disorder; substance-induced psychotic disorder; psychotic disorder due to another medical condition; paraphrenia (displaying symptoms similar to schizophrenia and starting late in life, when people are elderly). The most well-known psychotic disorder is schizophrenia and the majority of psychotic disorders display similar symptoms to schizophrenia.

Schizophrenia is a severe psychiatric disease with a heterogeneous course and symptom profile. Schizophrenia presents clinically with so-called positive and negative symptoms. The positive symptoms include delusions, hallucinations, disorganised speech, and disorganised or catatonic behaviours. Negative symptoms include affective flattening, restriction in the fluency and productivity of thought and speech and in the initiation of goal directed behaviour. The positive symptoms appear to reflect an excess or distortion of normal functions, whereas negative symptoms appear to reflect a diminution or loss of normal function. In addition, cognitive deficits (defects of working memory, information processing, attention/vigilance, learning, reasoning and social cognition) are common. Cognitive deficits generally show poor improvement with current antipsychotic treatment. Schizophrenic patients also suffer from mood symptoms. Besides these predominant symptoms, schizophrenia is associated with a comorbidity with other psychiatric symptoms such as manic and depressive symptoms, anxiety or obsessive-compulsive symptoms, substance abuse and dependence, and personality disorder.

According to the DMS-5, for the diagnosis of schizophrenia, a subject must have at least two of the following symptoms: delusions; hallucinations; disorganised speech; disorganised or catatonic behaviour and negative symptoms. At least one of the symptoms must be the presence of delusions, hallucinations or disorganised speech. Continuous signs of disturbance must persist for at least 6 months, during which the subject must experience at least 1 month of active symptoms, with social or occupational deterioration problems occurring over a significant amount of time.

Schizophrenia spectrum and psychotic disorders are psychiatric disorders that may develop or persist due to dysfunction of the microbiota-gut-brain axis. Therefore, in preferred embodiments, the compositions of the invention are for use in treating or preventing schizophrenia spectrum and/or psychotic disorders in a subject. In certain embodiments, the schizophrenia spectrum and psychotic disorder is selected from schizophrenia; schizoaffective disorder; schizophreniform disorder; brief psychotic disorder; delusional disorder; shared psychotic disorder; substance-induced psychotic disorder; psychotic disorder due to another medical condition and paraphrenia. In preferred embodiments, the compositions of the invention are for use in treating or preventing schizophrenia. In certain embodiments, the schizophrenia is selected from paranoid, disorganised, catatonic, undifferentiated and residual schizophrenia.

In certain embodiments, the compositions of the invention prevent, reduce or alleviate one or more of the symptoms of schizophrenia in a subject as classified by the DMS-5 criteria listed herein. These embodiments apply to the prevention, reduction or alleviation of symptoms of other schizophrenia spectrum and psychotic disorders. In certain embodiments, the compositions of the invention prevent, reduce or alleviate negative symptoms of schizophrenia. In certain embodiments, the compositions of the invention prevent, reduce or alleviate positive symptoms of schizophrenia. In certain embodiments, the compositions of the invention prevent, reduce or alleviate negative and positive symptoms of schizophrenia. In preferred embodiments, the compositions of the invention prevent, reduce or alleviate delusions, hallucinations, disorganised speech, and disorganised or catatonic behaviours in a subject with schizophrenia. In preferred embodiments, the compositions of the invention prevent, reduce or alleviate affective flattening, restriction in the fluency and productivity of thought and speech and in the initiation of goal directed behaviour in a subject with schizophrenia. In preferred embodiments, the compositions of the invention prevent, reduce or alleviate the cognitive defects and/or mood disorders in a subject with schizophrenia.

In certain embodiments, the compositions of the invention reduce the occurrence of positive and/or negative symptoms of schizophrenia in a subject within a 6 month period. In certain embodiments, the compositions of the invention improve social and/or occupational functionality in a subject with schizophrenia spectrum or psychotic disorder.

In some embodiments, the compositions of the invention improve the symptoms of schizophrenia spectrum or psychotic disorders according to a symptomatic or diagnostic scale. In certain embodiments, the scale for assessing symptomatic improvement is the Positive and Negative Symptom Scale (PANSS) and Brief Psychiatric Rating Scale (BPRS). In certain embodiments, the Scale for Assessment of Negative Symptoms (SANS) is used.

In some embodiments, the compositions of the invention improve the Clinical Global Impression - Global Improvement (CGI-I) scale for assessing psychiatric and neurological disorders. In some embodiments, the compositions of the invention display a positive effect on global social and occupational impairment of the subject with schizophrenia spectrum or psychotic disorders.

In preferred embodiments, the compositions of the invention prevent, reduce or alleviate at least one comorbidity of schizophrenia spectrum or psychotic disorder. In certain embodiments, the comorbidity is as manic and depressive symptoms, anxiety or obsessive-compulsive symptoms, substance abuse and dependence, and personality disorder.

In certain embodiments, the compositions of the invention are for use in treating or preventing treatment resistant of refractory schizophrenia.

In some embodiments, the compositions of the invention are particularly effective at preventing, reducing or alleviating schizophrenia spectrum or psychotic disorders when used in combination with another therapy for treating PTSD and stress disorders. In certain embodiments, such therapies include first generation antipsychotics including chlorpromazine, fluphenazine, haloperidol and/or perphenazine. In certain embodiments, such therapies include second generation therapies including aripiprazole (Abilify®); asenapine (Saphris®); brexpiprazole (Rexulti®); cariprazine (Vraylar®); clozapine (Clozaril®); iloperidone (Fanapt®); lurasidone (Latuda®); olanzapine (Zyprexa®); paliperidone (Invega); quetiapine (Seroquel®); risperidone (Risperdal®); ziprasidone (Geodon®).

### Bipolar disorder

Bipolar disorder in general is a chronic disease. Mania is the cardinal symptom of bipolar disorder. There are several types of bipolar disorder based upon the specific duration and pattern of manic and depressive episodes. In DMS-5, a distinction is made between bipolar I disorder, bipolar II disorder, cyclothymic disorder, rapid-cycling bipolar disorder and bipolar disorder NOS.

According to the DSM, mania is a distinct period of abnormally and persistently elevated, expansive, or irritable mood. The episode must last a week, and the mood must have at least three of the following symptoms: high self-esteem; reduced need for sleep; increase rate of speech; rapid jumping of ideas; easily distracted; an increased interest in goals or activities; psychomotor agitation; increased pursuit of activities with a high risk of danger.

Bipolar I disorder involves one or more manic or mixed (mania and depression) episodes and at least one major depressive episode (see above for symptoms of MDD episodes). Bipolar II disorder has one or more major depressive episodes accompanied by at least one hypomanic episode. There are no manic or mixed episodes. Hypomania is a lesser form of mania. The symptoms are responsible for significant social, occupational and functional impairments.

Cyclothymia is characterized by changing low-level depression along with periods of hypomania. The symptoms must be present for at least two years in adults or one year in children before a diagnosis can be made. Symptom free periods in adults and children last no longer than two months or one month, respectively. Rapid cycling bipolar disorder is a severe form of bipolar disorder. It occurs when a person has at least four episodes of major depression, mania, hypomania, or mixed states within a year. Not-otherwise specified (NOS) bipolar disorder classified bipolar symptoms that do not clearly fit into other types. NOS is diagnosed when multiple bipolar symptoms are present but not enough to meet the label for any of the other subtypes.

Bipolar disorder is associated with the following comorbidities: ADHD; anxiety disorders; substance disorders; obesity and metabolic syndrome.

Bipolar disorder is a psychiatric disorder that may develop or persist due to dysfunction of the microbiota-gut-brain axis. Therefore, in preferred embodiments, the compositions of the invention are for use in treating or preventing bipolar disorder in a subject. In certain embodiments, the bipolar disorder is bipolar I disorder. In certain embodiments, the bipolar disorder is bipolar II disorder. In certain embodiments, the bipolar disorder is cyclothymic disorder. In certain embodiments, the bipolar disorder is rapid-cycling bipolar disorder. In certain embodiments, the bipolar disorder is bipolar disorder NOS.

In preferred embodiments, the compositions of the invention prevent, reduce or alleviate one or more of the symptoms of bipolar disorder in a subject. In certain embodiments, the compositions of the invention prevent, reduce or alleviate the occurrence of manic episodes in a subject. In certain embodiments, the compositions of the invention prevent, reduce or alleviate the occurrence of an abnormally and persistently elevated, expansive, or irritable mood. In certain embodiments, the compositions of the invention prevent, reduce or alleviate one or more of the following symptoms: high self-esteem; reduced need for sleep; increase rate of speech; rapid jumping of ideas; easily distracted; an increased interest in goals or activities; psychomotor agitation; increased pursuit of activities with a high risk of danger. In certain embodiments, the compositions of the invention prevent, reduce or alleviate the occurrence of one or more manic or mixed episodes in a subject. In certain embodiments, the compositions of the invention reduce the occurrence of at least one major depressive episode in a subject. In certain embodiments, the compositions of the invention prevent, reduce or alleviate the occurrence of at least one major depressive episode accompanied by at least one hypomanic episode.

In preferred embodiments, the compositions of the invention treat the acute phase of bipolar disorder and/or prevent the occurrence of further episodes. In certain embodiments, the compositions of the invention treat the acute phase of manic/depressive episodes in a subject with bipolar disorder and prevent occurrence of further manic/depressive episodes.

In some embodiments, the compositions of the invention improve the symptoms of bipolar disorder according to a symptomatic or diagnostic scale. In certain embodiments, the scale for assessing symptomatic improvement of manic episodes is the Manic State Rating Scale and the Young Mania Rating Scale. In certain embodiments, the scale is the Bech-Rafaelsen Mania Scale (BRMAS). In certain embodiments, scales for assessing symptomatic improvement of the switch from manic to depressive episodes include the Hamilton Depression Rating Scale, the Montgomery-Asberg Rating Scale, and the Bech-Rafaelsen Depression Scale.

In some embodiments, the compositions of the invention improve the Clinical Global Impression - Global Improvement (CGI-I) scale for assessing psychiatric and neurological disorders. In some embodiments, the compositions of the invention display a positive effect on global social, occupational and functional impairments of the subject with bipolar disorder.

In preferred embodiments, the compositions of the invention prevent, reduce or alleviate at least one comorbidity of bipolar disorder. In certain embodiments, the comorbidity is selected from ADHD, anxiety disorders, substance disorder, obesity and metabolic syndrome.

In certain embodiments, the compositions of the invention are for use in treating or preventing manic-depressive illness and bipolar disorder unresponsive to lithium and divalproex.

In some embodiments, the compositions of the invention are particularly effective at preventing, reducing or alleviating bipolar disorder when used in combination with another therapy for treating bipolar disorder. In certain embodiments, such therapies include lithium carbonate, anticonvulsant drugs (including valproate, divalproex, carbamazepine and lamotrigine) and antipsychotic drugs (including aripiprazole, olanzapine, quetiapine and risperidone).

### Neurocognitive disorders and Alzheimer's disease

In DSM-5, the term dementia was replaced with the terms major neurocognitive disorder and mild neurocognitive disorder. Neurocognitive disorder is a heterogeneous class of psychiatric diseases. The most common neurocognitive disorder is Alzheimer's disease, followed by vascular dementias or mixed forms of the two. Other forms of neurodegenerative disorders (e.g. Lewy body disease, frontotemporal dementia, Parkinson's dementia, Creutzfeldt-Jakob disease, Huntington's disease, and Wernicke-Korsakoff syndrome) are accompanied by dementia.

The symptomatic criteria for dementia under DSM-5 are evidence of significant cognitive decline from a previous level of performance in one or more cognitive domains selected from: learning and memory; language; executive function; complex attention; perceptual-motor and social cognition. The cognitive deficits must interfere with independence in everyday activities. In addition, the cognitive deficits do not occur exclusively in the context of a delirium and are not better explained by another mental disorder (for example MDD or schizophrenia).

In addition to the primary symptom, subjects with neurocognitive disorders display behavioural and psychiatric symptoms including agitation, aggression, depression, anxiety, apathy, psychosis and sleep-wake cycle disturbances.

Neurocognitive disorders are psychiatric disorders that may develop or persist due to dysfunction of the microbiota-gut-brain axis. Therefore, in preferred embodiments, the compositions of the invention are for use in treating or preventing neurocognitive disorders in a subject. In preferred embodiments, the neurocognitive disorder is Alzheimer's disease. In other embodiments, the neurocognitive disorder is selected from vascular dementias; mixed form Alzheimer's disease and vascular dementia; Lewy body disease; frontotemporal dementia; Parkinson's dementia; Creutzfeldt-Jakob disease; Huntington's disease; and Wernicke-Korsakoff syndrome.

In preferred embodiments, the compositions of the invention prevent, reduce or alleviate one or more of the symptoms of neurocognitive disorders in a subject. In certain embodiments, the compositions of the invention prevent, reduce or alleviate the occurrence of cognitive decline in a subject. In certain embodiments, the compositions of the invention improve the level of performance of a subject with neurocognitive disorders in one or more cognitive domains selected from: learning and memory; language; executive function; complex attention; perceptual-motor and social cognition. In some embodiments, the compositions of the invention prevent, reduce or alleviate the occurrence of one or more behavioural and psychiatric symptoms associated with neurocognitive disorders selected from agitation, aggression, depression, anxiety, apathy, psychosis and sleep-wake cycle disturbances.

In certain embodiments, the compositions of the invention prevent, reduce or alleviate symptomatic disease by intervention in suspected pathogenic mechanisms at a preclinical stage. In certain embodiments, the compositions of the invention improve disease modification, with slowing or arrest of symptom progression. In some embodiments, the slowing or arrest of symptom progression correlates with evidence in delaying the underlying neuropathological process. In preferred embodiments, the compositions of the invention improve symptoms of neurocognitive disorders comprising enhanced cognitive and functional improvement. In preferred embodiments, the compositions of the invention improve the behavioural and psychiatric symptoms of dementia (BPSD). In preferred embodiments, the compositions of the invention improve the ability of a subject with neurocognitive disorder to undertake everyday activities.

In preferred embodiments, the compositions of the invention improve both cognition and functioning in a subject with Alzheimer's disease. In some embodiments, the composition of the invention improve the cognitive endpoint in a subject with Alzheimer's disease. In some embodiments, the compositions of the invention improve the functional endpoint in a subject with Alzheimer's disease. In preferred embodiments, the compositions of the invention improve the cognitive and functional endpoint in a subject with Alzheimer's disease. In yet further preferred embodiments, the compositions of the invention improve the overall clinical response (the global endpoint) in a subject with Alzheimer's disease.

In some embodiments, the compositions of the invention improve the symptoms of neurocognitive disorders according to a symptomatic or diagnostic test. In certain embodiments, the tests for assessing symptomatic improvement of Alzheimer's disease (and other neurocognitive disorders) are selected from objective cognitive, activities of daily living, global assessment of change, health related quality of life tests and tests assessing behavioural and psychiatric symptoms of neurocognitive disorders.

In certain embodiments, the objective cognitive tests for assessment of symptomatic improvement use the Alzheimer's disease Assessment Scale cognitive subscale (ADAS-cog) and the classic ADAS scale. In certain embodiments, symptomatic improvement of cognition is assessed using the Neurophysiological Test Battery for Use in Alzheimer's Disease (NTB).

In some embodiments, the global assessment of change test uses the Clinical Global Impression - Global Improvement (CGI-I) scale for assessing psychiatric and neurological disorders. In some embodiments, the global scale is the Clinician's Interview Based Impression of Change plus (CIBIC-plus). In some embodiments, the global scale is the Alzheimer's Disease Cooperative Study Unit Clinician's Global Impression of Change (ADCS-CGIC).

In certain embodiments, the health related quality of life measures are the Alzheimer's Disease-Related QOL (ADRQL) and the QOL-Alzheimer's Disease (QOL-AD).

In certain embodiments, the tests assessing behavioural and psychiatric symptoms of neurocognitive disorders are selected from the Behavioural pathology in Alzheimer's Disease Rating Scale (BEHAVE-AD); the Behavioural Rating Scale for Dementia (BRSD); the Neuropsychiatric Inventory (NPI); and the Cohen-Mansfield Agitation Inventory (CMAI).

In some embodiments, the compositions of the invention are particularly effective at preventing, reducing or alleviating neurocognitive disorders when used in combination with another therapy for treating neurocognitive disorders. In certain embodiments, such therapies include acetylcholinesterase inhibitors including donepezil (Aricept®), galantamine (Razadyne®) and rivastigmine (Exelon ®), and memantine.

### Parkinson's disease

Parkinson's disease is a common neurodegenerative disease neuropathologically characterised by degeneration of heterogeneous populations of neural cells (dopamine-producing cells). The clinical diagnosis of Parkinson's disease requires bradykinesia and at least one of the following core symptoms: resting tremor; muscle rigidity and postural reflex impairment. Other signs and symptoms that may be present or develop during the progression of the disease are autonomic disturbances (sialorrhoea, seborrhoea, constipation, micturition disturbances, sexual functioning, orthostatic hypotension, hyperhydrosis), sleep disturbances and disturbances in the sense of smell or sense of temperature. Depressive symptoms and cognitive dysfunction comorbidities develop in many Parkinson's disease patients, as well as neurocognitive disorders related to Lewy Bodies.

Parkinson's disease is a psychiatric disorder that may develop or persist due to dysfunction of the microbiota-gut-brain axis. Therefore, in preferred embodiments, the compositions of the invention are for use in treating or preventing Parkinson's disease in a subject.

In preferred embodiments, the compositions of the invention prevent, reduce or alleviate one or more of the symptoms of Parkinson's disease in a subject. In preferred embodiments, the compositions of the invention prevent, reduce or alleviate one or more core symptoms of Parkinson's disease in a subject. In certain embodiments, the compositions of the invention prevent, reduce or alleviate bradykinesia in a subject. In certain embodiments, the compositions of the invention prevent, reduce or alleviate resting tremor; muscle rigidity and/or postural reflex impairment in a subject. In certain embodiments, the compositions of the invention prevent, reduce or alleviate one or more symptoms associated with Parkinson's disease progression selected from autonomic disturbances (sialorrhoea, seborrhoea, constipation, micturition disturbances, sexual functioning, orthostatic hypotension, hyperhydrosis), sleep disturbances and disturbances in the sense of smell or sense of temperature.

In preferred embodiments, the compositions of the invention prevent, reduce or alleviate depressive symptoms comorbid with Parkinson's disease. In certain embodiments, the compositions of the invention improve verbal memory and/or executive functions. In certain embodiments, the compositions of the invention improve attention, working memory, verbal fluency and/or anxiety.

In other preferred embodiments, the compositions of the invention prevent, reduce or alleviate cognitive dysfunctions comorbid with Parkinson's disease.

In certain embodiments, the compositions of the invention prevent, reduce or alleviate Parkinson's disease progression. In certain embodiments, the compositions of the invention prevent, reduce or alleviate later motor complications. In certain embodiments, the compositions of the invention prevent, reduce or alleviate late motor fluctuations. In certain embodiments, the compositions of the invention prevent, reduce or alleviate neuronal loss. In certain embodiments, the compositions of the invention improve symptoms of Parkinson's disease dementia (PDD). In certain embodiments, the compositions of the invention prevent, reduce or alleviate impairment of executive function, attention and/or working memory. In certain embodiments, the compositions of the invention improve dopaminergic neurotransmission. In certain embodiments, the compositions of the invention prevent, reduce or alleviate impaired dopaminergic neurotransmission.

In some embodiments, the compositions of the invention improve the symptoms of Parkinson's disease according to a symptomatic or diagnostic scale. In certain embodiments, the tests for assessing symptomatic improvement of motor function in Parkinson's disease is the Unified Parkinson's Disease Rating Scale. In particular, UPDRS II considers the activity of daily life and UPDRS III considers motor-examination.

In some embodiments, the compositions of the invention improve the symptoms associated the PDD according to a symptomatic or diagnostic test and/or scale. In certain embodiments, the test or scale is selected from the Hopkins Verbal Learning Test - Revised (HVLT-R); the Delis-Kaplan Executive Function System (D-KEFS) Color-Word Interference Test; the Hamilton Depression Rating Scale (HAM-D 17; depression); the Hamilton Anxiety Rating Scale (HAM-A; anxiety) and the Unified Parkinson's Disease Rating Scale (UPDRS; PD symptom severity).

In some embodiments, the compositions of the invention improve the Clinical Global Impression - Global Improvement (CGI-I) scale for assessing psychiatric and neurological disorders. In some embodiments, the compositions of the invention display a positive effect on global social and occupational impairment of the subject with Parkinson's disease.

In some embodiments, the compositions of the invention are particularly effective at preventing, reducing or alleviating neurocognitive disorders when used in combination with another therapy for treating neurocognitive disorders. In certain embodiments, such therapies include dopamine agonists (including L-Dopa+); monoamine oxidase inhibitors, catecholamine-O-methyl transferase inhibitors; anticholinergics and glutamate modulators.

### Other central nervous system disorders

In preferred embodiments, the compositions of the invention are for use in treating or preventing a central nervous system disorder associated with dysfunction of the microbiota-gut-brain axis. In addition to the embodiments above, the compositions of the invention are for use in treating or preventing psychosis; chronic fatigue syndrome (myalgic encephalomyelitis) and/or chronic pain. In further embodiments, the compositions of the invention may be useful for treating or preventing motor neuron disease; Huntington's disease; Guillain-Barre syndrome and/or meningitis.

### Huntington's disease

Huntington's disease is an inherited brain condition, caused by an inherited faulty gene, which damages certain nerve cells in the brain. This brain damage gets progressively worse over time and can affect movement, cognition (perception, awareness, thinking, judgement) and behaviour. Early features of the disease can include personality changes, mood swings, fidgety movements, irritability and altered behaviour.

In certain embodiments, the compositions of the invention are for use in treating or preventing Huntington's disease. In certain embodiments, the compositions of the invention manage the symptoms of Huntington's disease, such as irritability or excessive movement. In certain embodiments, the compositions of the invention treat the depression associated with Huntington's disease and/or improve symptoms such as social withdrawal, lack or interest and sleep disturbance. In certain embodiments, the compositions of the invention improve memory and ability to concentrate on tasks. In certain embodiments, the compositions of the invention treat disabling abnormal movements. In certain embodiments, the compositions of the invention treat behavioural problems, antisocial behaviour, irritability and psychosis associated with Huntington's disease. In certain embodiments, the compositions of the invention induce neuroprotection and prevent nerve damage. In certain embodiments, the compositions of the invention increase the levels of dopamine and/or the levels of dopamine-containing cells.

### Neurochemical factors, neuropeptides and neurotransmitters and the microbiota-gut-brain axis

As outlined above, the microbiota-gut-brain axis is modulated by a number of different physiological systems. The microbiota-gut-brain axis is modulated by a number of signalling molecules. Alterations in the levels of these signalling molecules results in defects in central nervous system development and/or functionality. Indeed, many of the molecules disclosed in this section have been implicated in the functionality of the microbiota-gut-brain axis and the pathogenesis of central nervous system disorders or conditions ([14], [30], [10], [31]). The experiments performed by the inventors indicate that behavioural changes can be triggered by administration of *Clostridiaceae* strains. This effect may be mediated by an effect on levels of the signalling molecules, in particular those listed in this section. These alterations may be responsible for the therapeutic benefits associated with *Clostridiaceae* strains. Accordingly, due to the fact that the central nervous system disorders and conditions disclosed herein display a similar fundamental biochemical and physiological pathogenesis (*i.e.* via the microbiota-gut-brain axis), a similar therapeutic benefit of *Clostridiaceae* strains may be also achieved for these disorders and conditions. Administration of *Clostridiaceae* strains may be particularly effective for triggering behavioural changes associated with central nervous system disorders or conditions.

The signalling of the microbiota-gut-brain axis is modulated by levels of neurochemical factors, neuropeptides and neurotransmitters. Accordingly, in certain embodiments, the compositions of the invention modulates levels of neurochemical factors, neuropeptides and neurotransmitters. Accordingly, in certain preferred embodiments, the compositions of the invention directly alter CNS biochemistry. In preferred embodiments, the compositions of the invention modulate the levels of brain-derived neurotrophic factor (BDNF). In certain embodiments, the compositions of the invention modulate the levels of monoamines. In certain embodiments, the monoamines are serotonin (5-hydroxytryptamine (5-HT)), dopamine, norepinephrine and/or epinephrine. In certain embodiments, the monoamines are catecholamines. In certain embodiments, the catecholamines are dopamine, norepinephrine and epinephrine. In certain embodiments, the monoamines are tryptamines. In certain embodiments, the tryptamines are serotonin and melatonin. In certain embodiments, the compositions of the invention modulate the levels of acetylcholine.

In certain preferred embodiments, the compositions of the invention modulate the levels of oxytoxin. Oxytocin is associated with emotional, social, cognitive and neuroendocrine physiologies as well as autoregulation. In particular, oxytocin release is involved in anxiolysis; positive mood; maternal behaviour, pair bonding; sexual behaviour; social memory; olfactory memory; anorexiant effects; attenuation of the HPA axis response to stress; autoexcitation during birth and suckling as well as other physiological and psychological processes. In certain embodiments, the compositions of the invention increase the levels of oxytocin. In certain embodiments, the compositions of the invention decrease the levels of oxytocin. In certain embodiments, the compositions of the invention increase or decrease oxytocin signalling. In certain embodiments, the compositions of the invention modulate the levels of oxytocin receptors. In certain embodiments, the compositions of the invention modulate the flux of calcium ions into or out of neuronal, muscle and gastrointestinal cells. In preferred embodiments, the compositions of the invention treat and prevent neurodevelopmental and neuropsychiatric disorders and diseases associated with the microbiota-gut-brain axis by modulating the levels of oxytocin.

In certain embodiments, the compositions of the invention modulate the levels of brain monoamines and metabolites thereof. In preferred embodiments, the monoamine is serotonin. In certain embodiments, the compositions of the invention modulate the serotonergic and/or kynurenine routes of tryptophan metabolism. In certain embodiments, the compositions of the invention modulate the levels of serotonin metabolites, such as 5-Hydroxyindoleacetic acid (5-HIAA). In certain embodiments, the compositions of the invention modulate the levels of dopamine metabolites, such as Homovanillic acid (HVA). Modulation of these neurotransmitters and neurochemical factors is useful for treating stress, depression and anxiety-related disorders.

The signalling of the microbiota-gut-brain axis is modulated by levels of γ-aminobutyric acid (GABA). Accordingly, in preferred embodiments, the compositions of the invention modulate the levels of GABA. GABA is an inhibitory neurotransmitter that reduces neuronal excitability. In certain embodiments, the compositions of the invention increase the levels of GABA. In certain embodiments, the compositions of the invention decrease the levels of GABA. In certain embodiments, the compositions of the invention alter GABAergic neurotransmission. In certain embodiments, the compositions of the invention modulate the level of GABA transcription in different regions of the central nervous system. In certain embodiments, the commensal derived GABA crosses the blood-brain barrier and affects neurotransmission directly. In certain embodiments, the compositions of the invention lead to a reduction of GABA in the hippocampus, amygdala and/or locus coeruleus. In certain embodiments, the compositions of the invention lead to an increase of GABA in cortical regions.

The levels of neuroactive molecules, such as serotonin, melatonin, GABA, histamines and acetylcholine are linked to the pathophysiology of central nervous system diseases such as dementia, Alzheimer's disease and Huntington's disease.

The signalling of the microbiota-gut-brain axis is modulated by levels of histamines. Accordingly, in certain embodiments, the compositions of the invention modulate the levels of histamines. In certain embodiments, the histamines has an immunoregulatory effect. In certain embodiments, histamine levels enable translocation of bacteria from the lumen into systemic circulation. Therefore, in some embodiments, the compositions of the invention alter gastrointestinal tract permeability and/or barrier function. In certain other embodiments, the histamine acts as a neurotransmitter linked to central processes.

The signalling of the microbiota-gut-brain axis is modulated by the HPA axis. Accordingly, in certain embodiments, the compositions of the invention modulate HPA activity. In certain embodiments, the compositions of the invention attenuate the HPA stress response. In certain preferred embodiments, the compositions of the invention modulate inflammatory responses associated with HPA activity. In certain embodiments, the compositions of the invention modulate the levels of glucocorticoids. In certain preferred embodiments, the compositions of the invention modulate the levels of corticosterone and adrenaline. In certain embodiments, the compositions of the invention modulate the levels of corticotrophin-releasing factor and/or vasopressin. In certain embodiments, the compositions of the invention modulate the levels of vasopressin and/or other neurohypophysial or antidiuretic hormones. Alterations in HPA axis activity are associated with anxiety and stress disorders.

The signalling of the microbiota-gut-brain axis is modulated by alterations in the immune response and inflammatory factors and markers. Accordingly, in certain embodiments, the compositions of the invention may modulate the immune response. In certain embodiments, the compositions of the invention modulate the systemic levels of circulating neuroimmune signalling molecules. In certain preferred embodiments, the compositions of the invention modulate pro-inflammatory cytokine production and inflammation. In certain embodiments, the compositions of the invention modulate the inflammatory state. In certain embodiments, the compositions of the invention modulate the splenocyte proliferative response. In certain embodiments, the compositions of the invention modulate the systemic and/or plasma levels of C-reactive protein; IL-1 family cytokines; IL-1β; IL-2; IL-4; IL-6; IL-8; IL-10; IL-12p40; IL-17; IL-17A; IL-21; IL-23; TNF- α and IFN-γ. In some embodiments the compositions of the invention module the levels of anti-inflammatory cytokines, for example IL-10. In preferred embodiments, the compositions of the invention increase the levels of IL-10. In some embodiments, the compositions of the invention modulate the levels of TNF-α. In preferred embodiments, the compositions of the invention modulate the levels of IFN-γ. In some embodiments, the compositions of the invention modulate the IFN-γ:IL-10 ratio. In certain preferred embodiments, the compositions of the invention decrease the IFN-γ:IL-10 ratio. In preferred embodiments, the compositions of the invention decrease the levels of the pro-inflammatory cytokines TNF-α and IFN-γ. Increased circulating levels of cytokines are closely associated with various neuropsychiatric disorders, including depression, anxiety, schizophrenia and ASD. Evidence of inflammatory state alteration is highlighted in disorders such as schizophrenia, major depressive disorder and bipolar disorder.

In certain embodiments, the compositions of the invention modulates the levels of tolerance-mediating dendritic cells and reciprocally regulate pro and anti-inflammatory cytokine responses. In certain embodiments, the compositions of the invention decrease the systemic level of myeloperoxidase (a marker for inflammation and oxidation).Therapeutic modulators of the immune system and of inflammatory responses are useful for treating autism spectrum disorders and mood disorders.

In certain embodiments, the compositions of the invention modulate the immune response to an infection or vaccination. In certain embodiments, the compositions of the invention modulate the level of inflammation in response to infection or vaccination. In certain preferred embodiments, the compositions of the invention modulate maternal immune activation in response to an infection or vaccination during pregnancy. Accordingly, the compositions of the invention can be administered during pregnancy in order to treat or prevent a central nervous system disorder in the offspring.

The signalling of the microbiota-gut-brain axis is modulated by levels commensal metabolites. Accordingly, in certain embodiments, the compositions of the invention modulate the systemic levels of microbiota metabolites. In certain preferred embodiments, the compositions of the invention modulate the level of short chain fatty acids (SCFAs). In certain embodiments the level of SCFAs is increased or decreased. In some embodiments, the SCFA is butyric acid (BA) (or butyrate). In some embodiments, the SCFA is propionic acid (PPA). In some embodiments, the SCFA is acetic acid. In certain embodiments, the compositions of the invention modulate the ability of SCFAs to cross the blood-brain barrier. In certain embodiments, the compositions of the invention modulate the level of Polysaccharide A (PSA). In certain embodiments, the compositions of the invention modulate the levels of the potent pro-inflammatory endotoxin lipopolysaccharide (LPS). LPS leads to the production of inflammatory cytokines that alter physiological brain activity and modulate neuropeptide synthesis. LPS has an important influence on the modulation of the CNS, increasing the activity of areas devoted to the control of emotions (*e.g.* the amygdala). In certain embodiments, the compositions of the invention modulate the level of tryptophan and/or its metabolites. In certain embodiments, the compositions of the invention modulate the levels of 4-ethylphenylsulphate (4EPS; a uremic toxic associated with ASD-related behavioural abnormalities). In preferred embodiments, the compositions of the invention decrease the levels of 4-ethylphenylsulphate in a subject. The signals generated by the stimulation of neuronal signalling pathways caused by intraluminal gut stimuli strongly modulate brain activity, including pain perception, immune-response modulation, emotional control and other homeostatic functions. Accordingly, a composition able to modulate levels of these factors would have broad therapeutic applications for treating or preventing CNS disorders.

The signalling of the microbiota-gut-brain axis is modulated by levels gastrointestinal permeability. Accordingly, in some embodiments, the compositions of the invention alter the integrity of the gastrointestinal tract epithelium. In certain embodiments, the compositions of the invention modulate the permeability of the gastrointestinal tract. In certain embodiments, the compositions of the invention modulate the barrier function and integrity of the gastrointestinal tract. In certain embodiments, the compositions of the invention modulate gastrointestinal tract motility. In certain embodiments, the compositions of the invention modulate the translocation of commensal metabolites and inflammatory signalling molecules into the bloodstream from the gastrointestinal tract lumen.

The signalling of the microbiota-gut-brain axis is modulated by microbiome composition in the gastrointestinal tract. Accordingly, in certain embodiments, the compositions of the invention modulates the microbiome composition of the gastrointestinal tract. In certain embodiments, the compositions of the invention prevents microbiome dysbiosis and associated increases in toxic metabolites (*e.g.* LPS). In certain embodiments, the compositions of the invention modulate the levels of *Clostridium* in the gastrointestinal tract. In preferred embodiments, the compositions of the invention reduce the level of *Clostridium* in the gastrointestinal tract. In certain embodiments, the compositions of the invention reduce the levels of *Campylobacter jejuni.* In certain embodiments, the compositions of the invention modulate the proliferation of harmful anaerobic bacteria and the production of neurotoxins produced by these bacteria. In certain embodiments, the compositions of the invention modulate the microbiome levels of *Lactobacillus* and/or *Bifidobacterium.* In certain embodiments, the compositions of the invention modulate the microbiome levels of *Sutterella, Prevotella, Ruminoccucs* genera and/or the Alcaligenaceae family. In certain embodiments, the compositions of the invention increase the level of *Lactobacillus plantarum* and/or *Saccharomyces boulardii.*

In certain embodiments, the compositions of the invention prevent the dysregulation of the composition of the microbiome by extensive antibiotic use. In certain preferred embodiments, the compositions of the invention maintain a functional maternal microbiome composition upon administration of antibiotics during pregnancy. Accordingly, the compositions of the invention can be administered during pregnancy in order to treat or prevent a central nervous system disorder in the offspring.

Modulation of the microbiome has been shown to be effective at improving psychiatric disorder-related behaviours, including anxiety, depression, autism spectrum disorder, obsessive-compulsive disorder and memory abilities (including spatial and non-spatial memory), as well as other CNS-related disorders including Parkinson's disease. Certain studies have suggested that probiotics can reduce psychological stress, somatisation, depression and anger-hostility. The levels of *Lactobacillus* are associated with depression and have been implicated in pain signalling associated with gastrointestinal discomfort.

In certain embodiments, the compositions of the invention prevent, reduce or alleviate at least one of the behavioural symptoms associated with a central nervous system disorder described herein. In preferred embodiments, the compositions of the invention improve the overall clinical response in a subject.

In preferred embodiments, the compositions of the invention prevent, reduce or alleviate stereotyped, repetitive behaviour in a subject. In preferred embodiments, the compositions of the invention prevent, reduce or alleviate the occurrence of unusually restrictive behaviours and/or interests. In certain embodiments, the compositions of the invention prevent, reduce or alleviate recurrent obsessions and/or compulsions in a subject. In preferred embodiments, the compositions of the invention prevent, reduce or alleviate deficits in social behaviour in a subject. In preferred embodiments, the compositions of the invention prevent, reduce or alleviate avoidance behaviour in a subject. In preferred embodiments, the compositions of the invention prevent, reduce or alleviate deficits in communication behaviour in a subject.

In preferred embodiments, the compositions of the invention prevent, reduce or alleviate negative alterations in cognitions and mood in a subject. In preferred embodiments, the compositions of the invention prevent, reduce or alleviate anxiety-related behaviour in a subject. In preferred embodiments, the compositions of the invention prevent, reduce or alleviate stress-related behaviour in a subject. In preferred embodiments, the compositions of the invention prevent, reduce or alleviate depression-related behaviour in a subject. In preferred embodiments, the compositions of the invention prevent, reduce or alleviate aggressive behaviour in a subject. In preferred embodiments, the compositions of the invention prevent, reduce or alleviate the occurrence of an abnormally and persistently elevated, expansive, or irritable mood.

In preferred embodiments, the compositions of the invention prevent, reduce or alleviate intrusive thoughts in a subject. In preferred embodiments, the compositions of the invention prevent alterations in arousal and reactivity in a subject. In preferred embodiments, the compositions of the invention prevent, reduce or alleviate delusions, hallucinations, disorganised speech, and disorganised or catatonic behaviours in a subject. In preferred embodiments, the compositions of the invention prevent, reduce or alleviate affective flattening, restriction in the fluency and productivity of thought and speech and in the initiation of goal directed behaviour in a subject. In preferred embodiments, the compositions of the invention prevent, reduce or alleviate one or more of the following symptoms: high self-esteem; reduced need for sleep; increase rate of speech; rapid jumping of ideas; easily distracted; an increased interest in goals or activities; psychomotor agitation; increased pursuit of activities with a high risk of danger.

In preferred embodiments, the compositions of the invention improve spatial and/or non-spatial memory deficits in a subject. In preferred embodiments, the compositions of the invention improve both cognition and functioning in a subject. In preferred embodiments, the compositions of the invention improve locomotor activity in a subject. In preferred embodiments, the compositions of the invention prevent, reduce or alleviate bradykinesia in a subject. In preferred embodiments, the compositions of the invention prevent, reduce or alleviate resting tremor; muscle rigidity and/or postural reflex impairment in a subject.

In preferred embodiments, the compositions of the invention prevent, reduce or alleviate at least one comorbidity associated with a CNS disorder disclosed herein.

In preferred embodiments, the compositions of the invention improve the scores of a subject on at least one of the symptomatic and/or diagnostic scales for CNS disorders described herein. In certain other embodiments, the symptomatic and/or diagnostic scale is selected from the General Health Questionnaire (GHQ); the Depression Anxiety and Stress Scale (DASS); the Leiden Index of Depression Sensitivity-Revised (LEIDS-r); the Positive and Negative Symptom Scale (PANSS); the State-Trait Anxiety Inventory (STAI); the Development Behavior Checklist (DBC); the Beck Depression Inventory (BDI); the Beck Anxiety Inventory (BAI); the Hopkins Symptom Checklist (HSCL-90); the Hospital Anxiety and Depression Scale (HADS); the Perceived Stress Scale (PSS); the Coping Checklist (CCL) (also used to counter the stress of daily life); and the questionnaire-based Profile of Mood State (POMS).

In certain embodiments, the compositions of the invention may improve the symptomatic and/or diagnostic scale when assessing therapeutic efficacy in other animal models of CNS disorders known to a person skilled in the art. In addition to the behavioural assays disclosed in the examples, the compositions of the invention may improve reciprocal social interactions; olfactory communication; ultrasonic vocalisation; motor stereotypes (such as circling and vertical jumping), repetitive behaviour such as self-grooming and diffing; and perseverance in spatial tasks.

In addition, the compositions of the invention will be useful in treating and/or preventing CNS disorders in other animal models of CNS disorders. Other mouse models include inbred mice strains (including BALB/cJ and C58/J) and also genetically modified mice strains (including *NEUREXIN1, NEUROLIGIN3, NEUROLIGIN4, SHANK2, SHANK3, CNTNAP2, Tsc1*/*2* and *Fmr1* gene mutant mice strains).

In certain embodiments, the compositions of the invention improve social behaviour of a subject. In preferred embodiments, the compositions of the invention improve the recognition of social novelty in a subject. In preferred embodiments, the compositions of the invention improve the ability to discriminate between familiar and novel objects and familiar and novel subjects. In preferred embodiments, the composition of the invention improve ability to recognise other subjects.

In certain embodiments, the compositions of the invention improve depressive or depressive-like behaviour of a subject. In certain embodiments, the compositions of the invention improve learned helplessness in a subject.

In certain embodiments, the compositions of the invention modulate the levels of NMDA receptors and/or the subunits thereof. In certain embodiments, the compositions of the invention modulate the expression of the NMDA receptor 2A (also known and referred to herein as Grin2A). In certain embodiments, the compositions of the invention increase the expression of the NMDA receptor 2A. In certain embodiments, the compositions of the invention modulate the expression of the NMDA receptor 2B (also known and referred to herein as Grin2B). In certain embodiments, the compositions of the invention increase the expression of the NMDA receptor 2B. In certain embodiments, the compositions of the invention cause hypofunction of the NMDA receptor 2A. In certain embodiments, the compositions of the invention cause hyperfunction of the NMDA receptor 2A. Administration of NMDA receptor agonists was observed to improve learning and memory-related behaviour impairment in an olfactory bulbectomised mouse model of depression [32]. In certain embodiments, the compositions of the invention prevent, reduce or alleviate the symptoms of CNS disorders, for example impairment of learning and memory-related behaviour and/or depression, as a consequence of the modulation of NMDA receptor 2A and/or NMDA receptor 2B activity and/or expression. In preferred embodiments, the compositions of the inventions increase NMDA receptor activity and/or expression and improve learning and memory-related behaviour and/or depression.

In certain embodiments, the compositions of the invention modulate the expression of BDNF. In preferred embodiments, the compositions of the invention increase the expression of BDNF. In certain embodiments, the increase in BDNF is localised to the amygdala. In certain embodiments, the increase in BDNF is localised to the prefrontal cortex. BDNF and its receptor are essential for adult synaptic plasticity and the formation of memories [33]. A decrease in BDNF mRNA was observed in the hippocampus of individuals with Alzheimer's disease [34]. Meta-analysis studies also show that Alzheimer's disease patients have a reduced level of serum BDNF [35]. A decrease in BDNF protein was also observed in the caudate and putamen brain regions of patients suffering from Huntington's disease [36]. In preferred embodiments, the compositions of the invention prevent, reduce or alleviate the symptoms of CNS disorders, for example Alzheimer's and Huntington's disease, as a consequence of the increase in expression of BDNF.

In certain embodiments, the compositions of the invention modulate the level of noradrenaline. In preferred embodiments, the compositions of the invention increase the level of noradrenaline. In certain embodiments, the increase in noradrenaline is localised to the brainstem. A decrease in noradrenergic signalling is observed in Parkinson's disease patients [37]. Therapeutic agents which specifically increase noradrenaline activity are also used in the treatment of depression and ADHD (Attention-Deficit/Hyperactivity Disorder) [38]. In preferred embodiments, the compositions of the invention prevent, reduce or alleviate the symptoms of CNS disorders, for example Parkinson's disease, depression and ADHD, as a consequence of the increase in the level of noradrenaline.

In certain embodiments, the compositions of the invention modulate the level of serotonin. In preferred embodiments, the compositions of the invention decrease the level of serotonin. In certain embodiments, the decrease in noradrenaline is localised to the brainstem. Elevated levels of serum serotonin are commonly observed in autism spectrum disorder (ASD) [39]. In preferred embodiments, the compositions of the invention prevent, reduce or alleviate the symptoms of CNS disorders, for example ASD, as a consequence of the decrease in the level of serotonin.

### Modes of administration

Preferably, the compositions disclosed herein are to be administered to the gastrointestinal tract in order to enable delivery to and / or partial or total colonisation of the intestine with the bacterial strain of the invention. In other words, the bacteria may have colonised some or all of the gastrointestinal tract and / or such colonisation may be transient or permanent.

More specifically, in some embodiments, the "total colonisation of the intestine" means that bacteria have colonised all parts of the intestine (i.e. the small intestine, large intestine and rectum). Additionally or alternatively, the term "total colonisation" means that the bacteria engraft permanently in the some or all parts of the intestine.

In some embodiments, "partial colonisation of the intestine" means that bacteria have colonised some but not all parts of the intestine. Additionally or alternatively, the term "partial colonisation" means that the bacteria engraft transiently in some or all parts of the intestine.

The transience of engraftment can be determined by assessing (e.g. in a fecal sample) the abundance of the bacterial strain of the invention periodically (e.g. daily) following the end of a dosing interval to determine the washout period, i.e. the period between conclusion of the dosing interval and there being no detectable levels of the bacterial strain of the invention present. In embodiments of the invention, the washout period is 14 days or less, 12 days or less, 10 days or less, 7 days or less, 4 days or less, 3 days or less, 2 days or less or 1 day or less.

In embodiments of the invention, the bacteria of the present invention engraft transiently in the large intestine.

Generally, the compositions of the invention are administered orally, but they may be administered rectally, intranasally, or via buccal or sublingual routes.

In certain embodiments, the compositions of the invention may be administered as a foam, as a spray or a gel.

In certain embodiments, the compositions of the invention may be administered as a suppository, such as a rectal suppository, for example in the form of a theobroma oil (cocoa butter), synthetic hard fat (e.g. suppocire, witepsol), glycero-gelatin, polyethylene glycol, or soap glycerin composition.

In certain embodiments, the compositions of the invention are administered to the gastrointestinal tract via a tube, such as a nasogastric tube, orogastric tube, gastric tube, jejunostomy tube (J tube), percutaneous endoscopic gastrostomy (PEG), or a port, such as a chest wall port that provides access to the stomach, jejunum and other suitable access ports.

The compositions of the invention may be administered once, or they may be administered sequentially as part of a treatment regimen. In certain embodiments, the compositions of the invention are to be administered daily (either once or several times).

In certain embodiments, the compositions of the invention are administered regularly, such as daily, every two days, or weekly, for a period of time, such as for at least one week, two weeks, one month, two months, six months, or one year.

In some embodiments the compositions of the invention are administered for 7 days, 14 days, 16 days, 21 days or 28 days or no more than 7 days, 14 days, 16 days, 21 days or 28 days. For example, in some embodiments the compositions of the invention are administered for 16 days.

In certain embodiments of the invention, treatment according to the invention is accompanied by assessment of the patient's gut microbiota. Treatment may be repeated if delivery of and/or partial or total colonisation with the strain of the invention is not achieved such that efficacy is not observed, or treatment may be ceased if delivery and/or partial or total colonisation is successful, and efficacy is observed.

The compositions of the invention may be administered to a patient that has been identified as having an abnormal gut microbiota. For example, the patient may have reduced or absent colonisation by *Clostridiaceae.*

The compositions of the invention may be administered as a food product, such as a nutritional supplement.

Generally, the compositions of the invention are for the prevention or treatment of humans, although they may be used to treat animals including monogastric mammals such as poultry, pigs, cats, dogs, horses or rabbits. The compositions of the invention may be useful for enhancing the growth and performance of animals. If administered to animals, oral gavage may be used.

In some embodiments, the subject to whom the composition is to be administered is an adult human. In some embodiments, the subject to whom the composition is to be administered is an infant human.

### Compositions

The compositions of the invention comprise bacteria. In preferred embodiments of the invention, the composition is formulated in freeze-dried form. The composition of the invention may comprise granules or gelatin capsules, for example hard gelatin capsules, comprising a bacterial strain of the invention.

Preferably, the compositions of the invention comprise lyophilised bacteria. Lyophilisation of bacteria is a well-established procedure and relevant guidance is available in, for example, references [40-42]. The examples demonstrate that lyophilised compositions are particularly effective.

Alternatively, the compositions of the invention may comprise a live, active bacterial culture. The examples demonstrate that cultures of the bacteria of the invention are therapeutically effective.

In some embodiments, the bacterial strain in the composition of the invention has not been inactivated, for example, has not been heat-inactivated. In some embodiments, the bacterial strain in the composition of the invention has not been killed, for example, has not been heat-killed. In some embodiments, the bacterial strain in the composition of the invention has not been attenuated, for example, has not been heat-attenuated. For example, in some embodiments, the bacterial strain in the composition of the invention has not been killed, inactivated and/or attenuated. For example, in some embodiments, the bacterial strain in the composition of the invention is live. For example, in some embodiments, the bacterial strain in the composition of the invention is viable. For example, in some embodiments, the bacterial strain in the composition of the invention is capable of partially or totally colonising the intestine. For example, in some embodiments, the bacterial strain in the composition of the invention is viable and capable of partially or totally colonising the intestine.

In some embodiments, the composition comprises a mixture of live bacterial strains and bacterial strains that have been killed. Preferably, the composition comprises only *de minimis* levels of spores or does not comprise spores.

In preferred embodiments, the compositions of the invention are encapsulated to enable delivery of the bacterial strain to the intestine. Encapsulation protects the composition from degradation until delivery at the target location through, for example, rupturing with chemical or physical stimuli such as pressure, enzymatic activity, or physical disintegration, which may be triggered by changes in pH. Any appropriate encapsulation method may be used. Exemplary encapsulation techniques include entrapment within a porous matrix, attachment or adsorption on solid carrier surfaces, self-aggregation by flocculation or with cross-linking agents, and mechanical containment behind a microporous membrane or a microcapsule. Guidance on encapsulation that may be useful for preparing compositions of the invention is available in, for example, references [43-44].

The composition may be administered orally and may be in the form of a tablet, capsule or powder. Encapsulated products are preferred because *Clostridiaceae* are anaerobes.

A composition of the invention includes a therapeutically effective amount of a bacterial strain of the invention. A therapeutically effective amount of a bacterial strain is sufficient to exert a beneficial effect upon a patient. A therapeutically effective amount of a bacterial strain may be sufficient to result in delivery to and/or partial or total colonisation of the patient's intestine.

A suitable daily dose of the bacteria, for example for an adult human, may be from about 1 x 10³ to about 1 x 10¹¹ colony forming units (CFU); for example, from about 1 x 10⁷ to about 1 x 10¹⁰ CFU; in another example from about 1 x 10⁶ to about 1 x 10¹⁰ CFU; in another example from about 1 x 10⁷ to about 1 x 10¹¹ CFU; in another example from about 1 x 10⁸ to about 1 x 10¹⁰ CFU; in another example from about 1 x 10⁸ to about 1 x 10¹¹ CFU.

In certain embodiments, the dose of the bacteria is at least 10⁹ cells per day, such as at least 10¹⁰, at least 10¹¹, or at least 10¹² cells per day.

A dose of the composition may comprise the bacterial strain from about 1 x 10⁶ to about 1 x 10¹¹ colony forming units (CFU) /g, respect to the weight of the composition. The dose may be suitable for an adult human. For example, the composition may comprise the bacterial strain from about 1 x 10³ to about 1 x 10¹¹ CFU/g; for example, from about 1 x 10⁷ to about 1 x 10¹⁰ CFU/g; in another example from about 1 x 10⁶ to about 1 x 10¹⁰ CFU/g; in another example from about 1 x 10⁷ to about 1 x 10¹¹ CFU/g; in another example from about 1 x 10⁸ to about 1 x 10¹⁰ CFU/g; in another example from about 1 x 10⁸ to about 1 x 10¹¹ CFU/g, from about 1 x 10⁸ to about 1 x 10¹⁰ CFU/g. The dose may be, for example, 1g, 3g, 5g, and 10g.

The composition may be formulated as a probiotic. A probiotic is defined by the FAO/WHO as a live microorganism that, when administered in adequate amounts, confers a health benefit on the host.

Typically, a probiotic, such as the composition of the invention, is optionally combined with at least one suitable prebiotic compound. In certain embodiments, the probiotic composition of the present invention includes a prebiotic compound in an amount of from about 1 to about 30% by weight, respect to the total weight composition, (e.g. from 5 to 20% by weight). Known prebiotics include commercial products such as inulin and transgalacto-oligosaccharides.

A prebiotic compound is usually a non-digestible carbohydrate such as an oligo- or polysaccharide, or a sugar alcohol, which is not degraded or absorbed in the upper digestive tract. The carbohydrate may be selected from the group consisting of: fructo-oligosaccharides (or FOS), short-chain fructo-oligosaccharides, inulin, isomalt-oligosaccharides, pectins, xylo-oligosaccharides (or XOS), chitosan-oligosaccharides (or COS), beta-glucans, arable gum modified and resistant starches, polydextrose, D-tagatose, acacia fibers, carob, oats, and citrus fibers. In one aspect, the prebiotics are the short-chain fructo-oligosaccharides (for simplicity shown herein below as FOSs-c.c); said FOSs-c.c. are not digestible carbohydrates, generally obtained by the conversion of the beet sugar and including a saccharose molecule to which three glucose molecules are bonded.

Other prebiotic compounds (such as vitamin C, for example), may be included as oxygen scavengers and to improve the delivery and/or partial or total colonisation and survival *in vivo.* Alternatively, the probiotic composition of the invention may be administered orally as a food or nutritional product, such as milk or whey based fermented dairy product, or as a pharmaceutical product.

The compositions of the invention may comprise pharmaceutically acceptable excipients or carriers. Examples of such suitable excipients may be found in the reference [45]. Acceptable carriers or diluents for therapeutic use are well known in the pharmaceutical art and are described, for example, in reference [46]. Examples of suitable carriers include lactose, starch, glucose, methyl cellulose, magnesium stearate, mannitol, sorbitol and the like. Examples of suitable diluents include ethanol, glycerol and water. The choice of pharmaceutical carrier, excipient or diluent can be selected with regard to the intended route of administration and standard pharmaceutical practice. The pharmaceutical compositions may comprise as, or in addition to, the carrier, excipient or diluent any suitable binder(s), lubricant(s), suspending agent(s), coating agent(s), solubilising agent(s). Examples of suitable binders include starch, gelatin, natural sugars such as glucose, anhydrous lactose, free-flow lactose, beta-lactose, corn sweeteners, natural and synthetic gums, such as acacia, tragacanth or sodium alginate, carboxymethyl cellulose and polyethylene glycol. Examples of suitable lubricants include sodium oleate, sodium stearate, magnesium stearate, sodium benzoate, sodium acetate, sodium chloride and the like. Preservatives, stabilizers, dyes and even flavouring agents may be provided in the pharmaceutical composition. Examples of preservatives include sodium benzoate, sorbic acid, cysteine and esters of p-hydroxybenzoic acid, for example, in some embodiments the preservative is selected from sodium benzoate, sorbic acid and esters of p-hydroxybenzoic acid. Antioxidants and suspending agents may be also used. A further example of a suitable carrier is saccharose. A further example of a preservative is cysteine.

The compositions of the invention may be formulated as a food product. For example, a food product may provide nutritional benefit in addition to the therapeutic effect of the invention, such as in a nutritional supplement. Similarly, a food product may be formulated to enhance the taste of the composition of the invention or to make the composition more attractive to consume by being more similar to a common food item, rather than to a pharmaceutical composition. In certain embodiments, the composition of the invention is formulated as a milk-based product. The term "milk-based product" means any liquid or semi-solid milk- or whey-based product having a varying fat content. The milk-based product can be, e.g., cow's milk, goat's milk, sheep's milk, skimmed milk, whole milk, milk recombined from powdered milk and whey without any processing, or a processed product, such as yoghurt, curdled milk, curd, sour milk, sour whole milk, butter milk and other sour milk products. Another important group includes milk beverages, such as whey beverages, fermented milks, condensed milks, infant or baby milks, flavoured milks, ice cream, milk-containing food such as sweets.

In some embodiments, the compositions of the invention comprise one or more bacterial strains of the family *Clostridiaceae* and do not contain bacteria from any other family or comprise only *de minimis* or biologically irrelevant amounts of bacteria from another genus.

In certain embodiments, the compositions of the invention contain a single bacterial species and do not contain any other bacterial species. In certain embodiments, the compositions of the invention contain a single bacterial strain and do not contain any other bacterial strains. For example, the compositions of the invention may comprise bacteria only of the *Clostridiaceae* strain deposited under accession number NCIMB 43454. Such compositions may comprise only *de minimis* or biologically irrelevant amounts of other bacterial strains or species. Such compositions may be a culture that is substantially free from other species of organism. In some embodiments, such compositions may be a lyophilisate that is substantially free from other species.

In some embodiments, the compositions of the invention comprise more than one bacterial strain or species. For example, in some embodiments, the compositions of the invention comprise more than one (e.g. more than 1, 2, 3, 4, 5, 6, 7, 8, 9, 10, 15, 20, 25, 30, 35, 40 or 45) bacterial species. In some embodiments, the compositions of the invention comprise more than one (e.g. more than 1, 2, 3, 4, 5, 6, 7, 8, 9, 10, 15, 20, 25, 30, 35, 40 or 45) bacterial strains from the same species, and, optionally, do not contain bacteria from any other species. In some embodiments, the compositions of the invention comprise fewer than 50 (e.g. fewer than 45, 40, 35, 30, 25, 20, 19, 18, 17, 16, 15, 14, 13, 12, 11, 10, 9, 8, 7, 6, 5, 4 or 3 strains) species. In some embodiments, the compositions of the invention comprise fewer than 50 (e.g. fewer than 45, 40, 35, 30, 25, 20, 19, 18, 17, 16, 15, 14, 13, 12, 11, 10, 9, 8, 7, 6, 5, 4 or 3 strains) strains from within the same species, and, optionally, do not contain bacteria from any other species. In some embodiments, the compositions of the invention comprise 1-40, 1-30, 1-20, 1-19, 1-18, 1-17, 1-16, 1-15, 1-10, 1-9, 1-8, 1-7, 1-6, 1-5, 1-4, 1-3, 1-2, 2-50, 2-40, 2-30, 2-20, 2-19, 2-18, 2-17, 2-16, 2-15, 2-10, 2-5, 6-30, 6-15, 16-25, or 31-50 species. In some embodiments, the compositions of the invention comprise 1-40, 1-30, 1-20, 1-19, 1-18, 1-17, 1-16, 1-15, 1-10, 1-9, 1-8, 1-7, 1-6, 1-5, 1-4, 1-3, 1-2, 2-50, 2-40, 2-30, 2-20, 2-19, 2-18, 2-17, 2-16, 2-15, 2-10, 2-5, 6-30, 6-15, 16-25, or 31-50 strains from within the same species and, optionally, do not contain bacteria from any other species. In some embodiments, the compositions of the invention comprise more than one species from within the same family (e.g. more than 1, 2, 3, 4, 5, 6, 7, 8, 9, 10, 12, 15, 17, 20, 23, 25, 30, 35 or 40 species), and, optionally, do not contain bacteria from any other genus. In some embodiments, the compositions of the invention comprise less than 50 species from within the same family (e.g. less than 50, 45, 40, 35, 30, 25, 20, 15, 12, 10, 8, 7, 6, 5, 4 or 3 species), and, optionally, do not contain bacteria from any other genus. In some embodiments, the compositions of the invention comprise 1-50, 1-40, 1-30, 1-20, 1-15, 1-10, 1-9, 1-8, 1-7, 1-6, 1-5, 1-4, 1-3, 1-2, 2-50, 2-40, 2-30, 2-20, 2-15, 2-10, 2-5, 6-30, 6-15, 16-25, or 31-50 species from within the same family and, optionally, do not contain bacteria from any other genus. The invention comprises any combination of the foregoing.

In some embodiments, the composition comprises a microbial consortium. For example, in some embodiments, the composition comprises the *Clostridiaceae* bacterial strain as part of a microbial consortium. For example, in some embodiments, the *Clostridiaceae* bacterial strain is present in combination with one or more (e.g. at least 2, 3, 4, 5, 10, 15 or 20) other bacterial strains from the family *Clostridiaceae* and/or other genera. Preferably, these bacterial strains can live symbiotically *in vivo* in the intestine. For example, in some embodiments, the composition comprises the *Clostridiaceae* strain deposited under accession number NCIMB 43454 in combination with a bacterial strain from a different genus. In another example, the composition comprises the *Clostridiaceae* strain deposited under accession number NCIMB 43454 in combination with a bacterial strain from the family *Clostridiaceae* or the composition comprises the *Clostridiaceae* strain deposited under accession number NCIMB 43454 in combination with a bacterial strain from the family *Clostridiaceae* and a bacterial strain from a different genus. In some embodiments, the microbial consortium comprises two or more bacterial strains obtained from a faeces sample of a single organism, e.g. a human. In some embodiments, the microbial consortium is not found together in nature. For example, in some embodiments, the microbial consortium comprises bacterial strains obtained from faeces samples of at least two different organisms. In some embodiments, the two different organisms are from the same species, e.g. two different humans. In some embodiments, the two different organisms are an infant human and an adult human. In some embodiments, the two different organisms are a human and a non-human mammal. In some embodiments, the composition comprises fewer than 10, fewer than 9, fewer than 8, fewer than 7, fewer than 6, fewer than 5, fewer than 4, or fewer than 3 bacterial species.

In some embodiments, the composition of the invention additionally comprises a bacterial strain that has the same safety and therapeutic efficacy characteristics as the *Clostridiaceae* strain deposited under accession number NCIMB 43454, but which is not the *Clostridiaceae* strain deposited under accession number NCIMB 43454, or which is not *Clostridiaceae.*

In some embodiments in which the composition of the invention comprises more than one bacterial strain, species or genus, the individual bacterial strains, species or genera may be for separate, simultaneous or sequential administration. For example, the composition may comprise all of the more than one bacterial strain, species or genera, or the bacterial strains, species or genera may be stored separately and be administered separately, simultaneously or sequentially. In some embodiments, the more than one bacterial strains, species or genera are stored separately but are mixed together prior to use.

In some embodiments, the bacterial strain for use in the invention is obtained from human adult faeces. In some embodiments in which the composition of the invention comprises more than one bacterial strain, all of the bacterial strains are obtained from human adult faeces or if other bacterial strains are present, they are present only in *de minimis* amounts. The bacteria may have been cultured subsequent to being obtained from the human adult faeces and being used in a composition of the invention.

In some embodiments, the one or more *Clostridiaceae* bacterial strains is/are the only therapeutically active agent(s) in a composition of the invention. In some embodiments, the bacterial strain(s) in the composition is/are the only therapeutically active agent(s) in a composition of the invention.

The compositions for use in accordance with the invention may or may not require marketing approval.

In certain embodiments, the invention provides the above pharmaceutical composition, wherein said bacterial strain is lyophilised. In certain embodiments, the invention provides the above pharmaceutical composition, wherein said bacterial strain is spray dried. In certain embodiments, the invention provides the above pharmaceutical composition, wherein the bacterial strain is lyophilised or spray dried and wherein it is live. In certain embodiments, the invention provides the above pharmaceutical composition, wherein the bacterial strain is lyophilised or spray dried and wherein it is viable. In certain embodiments, the invention provides the above pharmaceutical composition, wherein the bacterial strain is lyophilised or spray dried and wherein it is capable of partially or totally colonising the intestine. In certain embodiments, the invention provides the above pharmaceutical composition, wherein the bacterial strain is lyophilised or spray dried and wherein it is viable and capable of partially or totally colonising the intestine.

In some cases, the lyophilised or spray dried bacterial strain is reconstituted prior to administration. In some cases, the reconstitution is by use of a diluent described herein.

The compositions of the invention can comprise pharmaceutically acceptable excipients, diluents or carriers.

In certain embodiments, the invention provides a pharmaceutical composition comprising: a bacterial strain as discussed earlier; and a pharmaceutically acceptable excipient, carrier or diluent; wherein the bacterial strain is in an amount sufficient to treat a disorder when administered to a subject in need thereof; and wherein the disorder is sensory hypersensitivity, such as sensory hypersensitivity associated with neuropathy, complex regional pain syndrome, postherpetic neuralgia, fibromyalgia, or migraine. Preferably, the disorder is fibromyalgia. The disorder may be allodynia and/or hyperalgesia, such as allodynia and/or hyperalgesia associated with neuropathy, complex regional pain syndrome, postherpetic neuralgia, fibromyalgia, or migraine. Preferably, the disorder is fibromyalgia.

In certain embodiments, the invention provides the above pharmaceutical composition, wherein the amount of the bacterial strain is from about 1 × 10³ to about 1 × 10¹¹ colony forming units per gram with respect to a weight of the composition.

In certain embodiments, the invention provides the above pharmaceutical composition, wherein the composition is administered at a dose of 1 g, 3 g, 5 g or 10 g.

In certain embodiments, the invention provides the above pharmaceutical composition, wherein the composition is administered by a method selected from the group consisting of oral, rectal, subcutaneous, nasal, buccal, and sublingual.

In certain embodiments, the invention provides the above pharmaceutical composition, comprising a carrier selected from the group consisting of lactose, starch, glucose, methyl cellulose, magnesium stearate, mannitol and sorbitol.

In certain embodiments, the invention provides the above pharmaceutical composition, comprising a diluent selected from the group consisting of ethanol, glycerol and water.

In certain embodiments, the invention provides the above pharmaceutical composition, comprising an excipient selected from the group consisting of starch, gelatin, glucose, anhydrous lactose, free-flow lactose, beta-lactose, corn sweetener, acacia, tragacanth, sodium alginate, carboxymethyl cellulose, polyethylene glycol, sodium oleate, sodium stearate, magnesium stearate, sodium benzoate, sodium acetate and sodium chloride.

In certain embodiments, the invention provides the above pharmaceutical composition, further comprising at least one of a preservative, an antioxidant and a stabilizer.

In certain embodiments, the invention provides the above pharmaceutical composition, comprising a preservative selected from the group consisting of sodium benzoate, sorbic acid and esters of p-hydroxybenzoic acid.

In certain embodiments, the invention provides the above pharmaceutical composition, wherein said bacterial strain is lyophilised.

In certain embodiments, the invention provides the above pharmaceutical composition, wherein when the composition is stored in a sealed container at about 4°C or about 25°C and the container is placed in an atmosphere having 50% relative humidity, at least 80% of the bacterial strain as measured in colony forming units, remains after a period of at least about: 1 month, 3 months, 6 months, 1 year, 1.5 years, 2 years, 2.5 years or 3 years.

In some embodiments, the composition of the invention is provided in a sealed container comprising a composition as described herein. In some embodiments, the sealed container is a sachet or bottle. In some embodiments, the composition of the invention is provided in a syringe comprising a composition as described herein.

The composition of the present invention may, in some embodiments, be provided as a pharmaceutical formulation. For example, the composition may be provided as a tablet or capsule. In some embodiments, the capsule is a gelatine capsule ("gel-cap"). The capsule can be a hard or a soft capsule. In some embodiments, the formulation is a soft capsule. Soft capsules are capsules which may, owing to additions of softeners, such as, for example, glycerol, sorbitol, maltitol and polyethylene glycols, present in the capsule shell, have a certain elasticity and softness. Soft capsules can be produced, for example, on the basis of gelatine or starch. Gelatine-based soft capsules are commercially available from various suppliers. Depending on the method of administration, such as, for example, orally or rectally, soft capsules can have various shapes, they can be, for example, round, oval, oblong or torpedo-shaped. Soft capsules can be produced by conventional processes, such as, for example, by the Scherer process, the Accogel process or the droplet or blowing process.

In some embodiments, the compositions of the invention are administered orally. Oral administration may involve swallowing, so that the compound enters the gastrointestinal tract.

Pharmaceutical formulations suitable for oral administration include solid plugs, solid microparticulates, semi-solid and liquid (including multiple phases or dispersed systems) such as tablets; soft or hard capsules containing multi- or nano-particulates, liquids (e.g. aqueous solutions), emulsions or powders; lozenges (including liquid-filled); chews; gels; fast dispersing dosage forms; films; ovules; sprays; and buccal/mucoadhesive patches.

In some embodiments the pharmaceutical formulation is an enteric formulation, i.e. a gastro-resistant formulation (for example, resistant to gastric pH) that is suitable for delivery of the composition of the invention to the intestine by oral administration. Enteric formulations may be particularly useful when the bacteria or another component of the composition is acid-sensitive, e.g. prone to degradation under gastric conditions.

In some embodiments, the enteric formulation comprises an enteric coating. In some embodiments, the formulation is an enteric-coated dosage form. For example, the formulation may be an enteric-coated tablet or an enteric-coated capsule, or the like. The enteric coating may be a conventional enteric coating, for example, a conventional coating for a tablet, capsule, or the like for oral delivery. The formulation may comprise a film coating, for example, a thin film layer of an enteric polymer, e.g. an acid-insoluble polymer.

In some embodiments, the enteric formulation is intrinsically enteric, for example, gastro-resistant without the need for an enteric coating. Thus, in some embodiments, the formulation is an enteric formulation that does not comprise an enteric coating. In some embodiments, the formulation is a capsule made from a thermogelling material. In some embodiments, the thermogelling material is a cellulosic material, such as methylcellulose, hydroxymethylcellulose or hydroxypropylmethylcellulose (HPMC). In some embodiments, the capsule comprises a shell that does not contain any film forming polymer. In some embodiments, the capsule comprises a shell and the shell comprises hydroxypropylmethylcellulose and does not comprise any film forming polymer (e.g. see [47]). In some embodiments, the formulation is an intrinsically enteric capsule (for example, Vcaps® from Capsugel).

### Culturing methods

The bacterial strains for use in the present invention can be cultured using standard microbiology techniques as detailed in, for example, references [48-50].

The solid or liquid medium used for culture may for example be YCFA agar or YCFA medium. YCFA medium may include (per 100ml, approximate values): Casitone (1.0 g), yeast extract (0.25 g), NaHCO₃ (0.4 g), cysteine (0.1 g), K₂HPO₄ (0.045 g), KH₂PO₄ (0.045 g), NaCl (0.09 g), (NH₄)₂SO₄ (0.09 g), MgSO₄ · 7H₂O (0.009 g), CaCl₂ (0.009 g), resazurin (0.1 mg), hemin (1 mg), biotin (1 µg), cobalamin (1 µg), *p*-aminobenzoic acid (3 µg), folic acid (5 µg), and pyridoxamine (15 µg).

### General

The practice of the present invention will employ, unless otherwise indicated, conventional methods of chemistry, biochemistry, molecular biology, immunology and pharmacology, within the skill of the art. Such techniques are explained fully in the literature. See, *e.g.,* references [51- 58], *etc.*

The term "comprising" encompasses "including" as well as "consisting" *e.g.* a composition "comprising" X may consist exclusively of X or may include something additional *e.g.* X + Y.

The term "about" in relation to a numerical value *x* is optional and means, for example, x+10%.

The word "substantially" does not exclude "completely" *e.g.* a composition which is "substantially free" from Y may be completely free from Y. Where necessary, the word "substantially" may be omitted from the definition of the invention.

References to a percentage sequence identity between two nucleotide sequences means that, when aligned, that percentage of nucleotides are the same in comparing the two sequences. This alignment and the percent homology or sequence identity can be determined using software programs known in the art, for example those described in section 7.7.18 of ref. [59]. A preferred alignment is determined by the Smith-Waterman homology search algorithm using an affine gap search with a gap open penalty of 5 and a gap extension penalty of 2, BLOSUM matrix of 62. The Smith-Waterman homology search algorithm is disclosed in ref. [60].

Unless specifically stated, a process or method comprising numerous steps may comprise additional steps at the beginning or end of the method, or may comprise additional intervening steps. Also, steps may be combined, omitted or performed in an alternative order, if appropriate.

Various embodiments of the invention are described herein. It will be appreciated that the features specified in each embodiment may be combined with other specified features, to provide further embodiments. In particular, embodiments highlighted herein as being suitable, typical or preferred may be combined with each other (except when they are mutually exclusive).

All patent and literature references cited in the present specification are hereby incorporated by reference in their entirety.

Any reference to a method for treatment comprising administering an agent to a patient, also covers that agent for use in said method for treatment, as well as the use of the agent in said method for treatment, and the use of the agent in the manufacture of a medicament.

The following examples are offered for illustrative purposes only, and are not intended to limit the scope of the present invention in any way.

### MODES FOR CARRYING OUT THE INVENTION

The following examples demonstrate the therapeutic efficacy of bacterial strains of the family *Clostridiaceae.* As also discussed above, in general, a therapeutic effect does not mean a complete cure of the disease necessarily. Rather, a therapeutic effect may also be given where one or more symptoms of the disease are ameliorated or cured.

### Example 1 - Effect of Clostridiaceae on intestinal permeability

### Summary

This study investigated the effect of *Clostridiaceae* strain NCIMB 43454 on intestinal permeability of the ileum. Excessive permeability, or 'leakiness', of the intestine is associated with a number of CNS disorders such as autism and Parkinson's disease.

### Materials and Methods

### Method

Male BALB/c mice received oral gavage (200µL volume) of 1 x 10⁹ CFU of *Clostridiaceae* strain NCIMB 43454 for 6 consecutive days. On day 7, the animals were euthanized by cervical dislocation, and the distal ileum was removed, placed in chilled Krebs solution, opened along the mesenteric line and carefully rinsed. Preparations were then placed in Ussing chambers (Harvard Apparatus, Kent, UK, exposed area of 0.12 cm²) as described previously (Hyland and Cox, 2005) with oxygenated (95% O₂, 5% CO₂) Krebs buffer maintained at 37°C. 4 kDa FITC-dextran was added to the mucosal chamber at a final concentration of 2.5 mg/mL; 200 µL samples were collected from the serosal chamber every 30 min for the following 3 h and fluorescence in those samples measured.

### Results

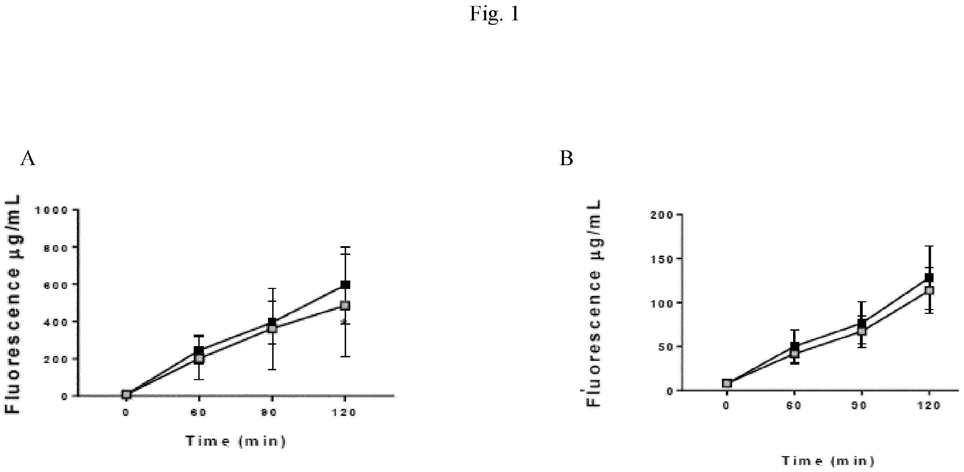

As shown in Fig. 1, NCIMB 43454 decreased the permeability of the ileum. Therefore, bacterial strains of the invention may be useful in modifying ileal permeability and thus in the treatment or prevention of disorders or conditions associated with ileal permeability.

### Example 2 - Effect of Clostridiaceae on expression of Tryptophan hydroxylase-1

### Summary

This study investigated the effect of *Clostridiaceae* strain NCIMB 43454 on the expression of Tryptophan hydroxylase-1. Tryptophan hydroxylase-1 (TPH-1) catalyzes the formation of 5-hydroxy-L-tryptophan (5-HTP) from L-tryptophan, the first and rate-limiting step in the biosynthesis of 5-HT. TPH-1 deficiency is associated with a number of conditions, for example, allograft tolerance, induction of tumour remission, and neuroinflammation.

### Materials and Methods

### Method

Male BALB/c mice received oral gavage (200µL volume) of 1 x 10⁹ CFU of *Clostridiaceae* strain NCIMB 43454 for 6 consecutive days. On day 7, the animals were euthanized. Intestinal tissue (1 cm segments of ileum) was excised. Total RNA was extracted using the mirVana™ miRNA Isolation kit (Ambion/Llife technologies, Paisley, UK) and DNase treated (Turbo DNA-free, Ambion/life technologies) according to the manufacturer's recommendations. RNA was quantified using a NanoDrop™ spectrophotometer (Thermo Fisher Scientific Inc., Wilmington, Delaware, USA) according to the manufacturer's instructions. RNA quality was assessed using the Agilent Bioanalyzer (Agilent, Stockport, UK) according to the manufacturer's instructions and an RNA integrity number (RIN) was calculated. RNA with an RIN value >7 was used for subsequent experiments. RNA was reverse transcribed to cDNA using the Applied Biosystems High Capacity cDNA kit (Applied Biosystems, Warrington, UK) according to manufacturer's instructions. Briefly, Multiscribe Reverse Transcriptase (50 U/(µL) was added as part of RT master mix, incubated for 25°C for 10 min, 37°C for 2 h, 85°C for 5 min and stored at 4°C. Quantitative PCR was carried out using probes (6 carboxy fluorescein - FAM) designed by Applied Biosystems to mouse specific targeted genes, while using β-actin as an endogenous control. Amplification reactions contained 1 µl cDNA, 5 µl of the 2X PCR Master mix (Roche), and 900 nM of each primer and were brought to a total of 10 µl by the addition of RNase-free water. All reactions were performed in triplicate using 96-well plates on the LightCycler™ 480 System. Thermal cycling conditions were as recommended by the manufacturer (Roche) for 55 cycles. To check for amplicon contamination, each run contained no template controls in triplicate for each probe used. Cycle threshold (Ct) values were recorded. Data was normalized using β-actin and transformed using the 2-ΔΔCT method and presented as a fold change vs. control group.

### Results

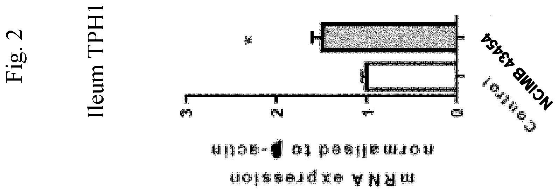

As shown in Fig. 2, NCIMB 43454 effected a significant increase in TPH1 expression in the ileum.

This study shows that *Clostridiaceae* may be useful in the treatment or prevention of disorders or conditions associated with dysregulated or otherwise abnormal expression of tryptophan hydroxylase-1 in the ileum.

### Example 3 - Effect of Clostridiaceae on short-chain fatty acid production

### Summary

This study investigated the effect of *Clostridiaceae* strain NCIMB 43454 on the production of short-chain fatty acids (SCFAs) in mice. SCFAs, which include acetate, propionate, valerate, butyrate, isobutyrate and isovalerate are microbial byproducts of dietary fibre. An increase in any SCFA suggests an increase in productivity of the microbiota and is a desirable trait.

### Materials and methods

### Method

Male BALB/c mice received oral gavage (200µL volume) of 1 X 10⁹ CFU of *Clostridiaceae* strain NCIMB 43454 for 6 consecutive days. On day 7, the animals were euthanized. The caecum was removed, weighed and stored at -80 °C for SCFAs analysis. Caecum content was mixed and vortexed with MilliQ water and incubated at room temperature for 10 min. Supernatants were obtained by centrifugation (10000 g, 5 min, 4 °C) to pellet bacteria and other solids and filtration by 0.2µm. It was transferred to a clear GC vial and 2-Ethylbutyric acid (Sigma) was used as the internal standard. The concentration of SCFA was analyzed using a Varian 3500 GC flame-ionization system, fitted with a with a ZB-FFAP column (30 m x 0.32 mm x 0.25 mm; Phenomenex). A standard curve was built with different concentrations of a standard mix containing acetate, propionate, iso-butyrate, n-butyrate, isovalerate and valerate (Sigma). Peaks were integrated by using the Varian Star Chromatography Workstation version 6.0 software. All SCFA data are expressed as µmol/g.

### Results

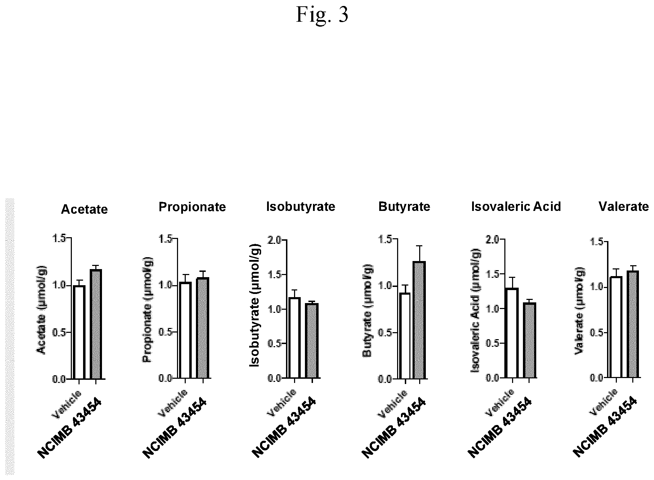

Figure 3 shows production of SCFAs following treatment with *Clostridiaceae* strain NCIMB 43454. Increased production of acetate, butyrate and propionate were observed. This suggests that *Clostridiaceae* may contribute to SCFAs production, and therefore be useful in the treatment or prevention of disorders or conditions associated with a reduced productivity of the microbiota.

### Example 4 - Effect of Clostridiaceae on gene expression in the brain

### Summary

This study investigated the effect of *Clostridiaceae* strain NCIMB 43454 on expression of certain genes of interest in the brain. mRNA levels for markers for the oxytocinergic system (oxytocin receptor and vasopressin receptor), endocrine system (mineralocorticoid (Nr3c1); glucocorticoid receptor (Nr3c2); corticosterone releasing factor (CRF) and receptors; Brain derived neurotrophic factor (BDNF)), immune system (Il-6, TNF-α, TLR-4); and neurotransmitter systems (NMDA receptor 2A (Grin2A); NMDA receptor 2B (Grin2B); GABAA receptor subunit A2; GABAB receptor subunit B1; serotonin 2C) were assessed in the amygdala, hippocampus and prefrontal cortex (PFC), which are key brain regions of the limbic system involved in emotional response.

### Materials and Methods

### Method

Male BALB/c mice received oral gavage (200µL volume) of 1 x 10⁹ CFU of *Clostridiaceae* strain NCIMB 43454 for 6 consecutive days. On day 7, the animals were euthanized. The brain was quickly excised, dissected and each brain region was snap-frozen on dry ice and stored at -80 °C for further analysis. mRNA expression was quantified as described in Example 2.

### Results

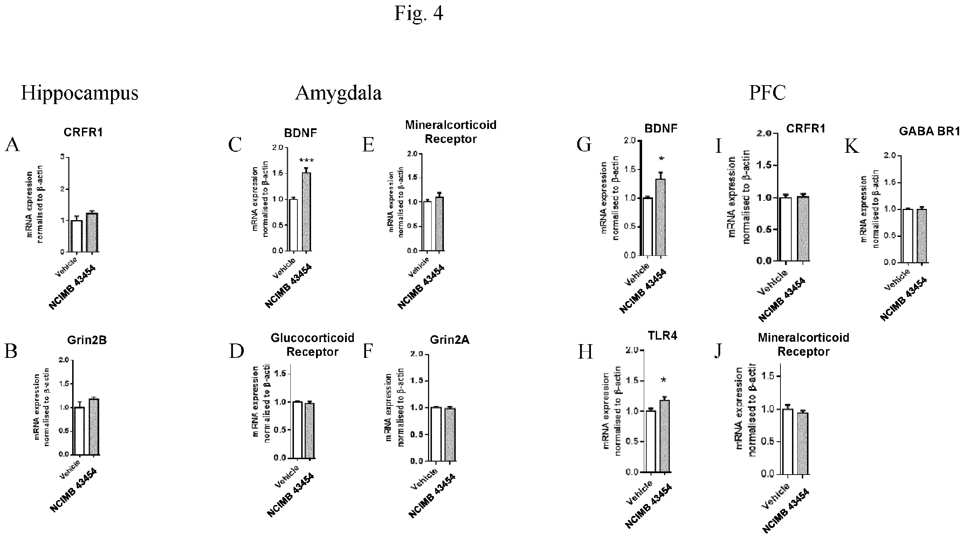

As shown in Figure 4, a significant change in the expression of CRFR1 (A) and Grin2b (B) in the hippocampus; BDNF (C), glucocorticoid receptor (D), mineralocorticoid receptor (E) and Grin2a (F) in the amygdala; and BDNF (G), TLR4 (H), CRFR1 (I), mineralocorticoid receptor (J) and GABA BR1 (K) in the PFC was observed in mice which have been treated with *Clostridiaceae* strain NCIMB 43454, relative to treatment with vehicle only. This indicates that *Clostridiaceae* may be useful in the treatment or prevention of disorders or conditions that may benefit from the modulation in the levels of expression of these proteins in the brain, e.g. CNS diseases and disorders.

### Example 5 - Effect of Clostridiaceae on behaviour in models of autism spectrum disorders

### Summary

This study investigated the effect of *Clostridiaceae* strain NCIMB 43454 on a number of behavioural readouts including anxiety-related behaviour (elevated plus maze test), social behaviour (3-chamber social interaction test), cognitive performance (novel object recognition) and depression/acute stress (forced swim test) in both an environmental animal model (maternal immune activation model) and a genetic animal model (Btbr mouse strain) of autism spectrum disorder.

### Materials and Methods

### BTBR mouse model

Btbr animals were bred in house with brother-sister mating. The male offspring from these animals were separated from their mothers at 3 weeks old and daily administration of the live biotherapeutic or control commenced at 8 weeks of age. Behavioural assessments started once the animals reached 11 weeks old. A control age-matched C57/B16 group was included as a reference control group.

### MIA mouse model

Female C57/B16 mice (8 weeks old) and age matched males were purchased from Harlan UK. After 1 week of habituation these animals were mated. At embryonic day 12.5, females received either an injection of the viral mimetic poly-IC to activate the maternal immune system, or a saline vehicle injection. The male offspring from these animals were separated from their mothers at 3 weeks old and daily administration of the live biotherapeutic or control commenced at 8 weeks of age. Behavioural assessments started once the animals reached 11 weeks old.

### Elevated plus maze

The set up was made of a grey plastic cross-shaped maze 1 m elevated from the floor, comprising two open (fearful) and two closed (safe) arms (50 × 5 × 15 cm walls or 1 cm no wall). Experiments occurred under red light (~5 lux). Mice were individually placed into the centre of the maze facing an open arm (to avoid direct entrance into a closed one) and were allowed 5-min free exploration. Experiments were videotaped using a ceiling camera for further parameters analysis using Ethovision software (3.1 version, Noldus, TrackSys, Nottingham, UK). The percentage of time spent, distance moved and the number of entries in each arm were measured, for anxiety behaviour and locomotor activity, respectively (entrance in an arm was defined as all four paws inside the arm).

### Social behaviour

The social testing apparatus was a rectangular, three-chambered box. Each chamber was 20 cm L × 40 cm W × 22 cm H. Dividing walls were made with small circular openings (5 cm in diameter) allowing access into each chamber. Two identical wire cup-like cages, with a bottom diameter of 10 cm, 13 cm in height and bars spaced 1.2 cm, allowing nose contact between the bars, but preventing fighting, were placed inside each side chamber in bilaterally symmetric positions. The test has three phases of 10 min each: 1) habitation 2) mouse versus object 3) novel mouse versus familiar mouse. Experiments were videotaped using a ceiling camera for further parameters analysis using Ethovision software (3.1 version, Noldus, TrackSys, Nottingham, UK). For the first phase the test mouse was placed into the middle chamber and allowed to explore the entire box with an empty small wire cages inside for a 10-min habituation session. After the habituation period, the test mouse was removed from the testing box for a short interval while an object was placed in one side chamber and an unfamiliar conspecific male mouse (no prior contact with the test subject) in the other side chamber, both enclosed in a wire cup-like cage. During phase two, the test mouse was placed in the middle chamber and allowed to explore the entire box for 10 min. The amount of time spent exploring the object or mouse in each chamber and the number of entries into each chamber were evaluated. The location of the unfamiliar mouse in the left vs right side chamber was systematically alternated between trials. An entry was defined as all four paws in one chamber. During the third phase an object was replaced with an unfamiliar mouse serving as a novel mouse and in the other chamber the mouse used in phase two was kept the same, now serving as familiar mouse. After every trial, all chambers and cup-like wire cages were cleaned with 10% ethanol, dried and ventilated for a few minutes to prevent olfactory cue bias and to ensure proper disinfection. Lack of innate side preference was confirmed during the initial 10 min of habituation to the entire arena. Control animals are naturally interested in a conspecific mouse more than an inanimate object (sociability). In a similar vein, control animals spend more time interacting with a novel unfamiliar mouse then one they have already had interactions with. Some animal models, as used here, have deficits in this social paradigm.

### Novel object recognition

Mice were placed in the middle of a grey plastic rectangular box (40 × 32 × 23 cm, L × W × H) under a dimly light, 60 lux at the level of the arena, for 10 minutes. 24 hours after mice were placed in the box with the two identical objects for a total time of 10 min (acquisition phase). After 24 hours, one of the two identical objects was substituted with a novel object and mice were placed in the middle of the box at the mid-point of the wall opposite the sample objects for a total time of 10 minutes (retention phase). Animals were acclimatized to the testing room for 30 minutes prior to each experiment. Box and objects were cleaned with 10% alcohol to avoid any cue smell between each trial. Experiments were videotaped using a ceiling camera for further parameter analysis. Directed contacts with the objects, include any contact with mouth, nose or paw or minimal defined distance were counted as an interaction. Control animals will discriminate between an object they have had time to explore and a new object.

### Forced swim

Mice were individually placed in a clear glass cylinder (24 × 21 cm diameter), containing 15-cm-depth water (25 ± 0.5 °C). Water was changed between each animal. The test lasted 6 minutes and experiments were videotaped using a numeric tripod-fixed camera; data were further scored twice using the videos (Video Media Player software) and averaged by an experimenter blind to conditions. The latency to immobility was scored. The time of immobility (s) was measured for the last 4 minutes of the test, with immobility being defined as a total absence of movement except slight motions to maintain the head above the water. An increase in immobility means an increase in depressive-like behaviour as the animal has resigned itself to its situation (learned helplessness).

### Method

From 8 weeks of age, animals received daily oral gavage of PBS or the live biotherapeutics prepared to 1 x 10⁹ CFU/mL in PBS. Dosing continued daily throughout the behavioural assessment. Behavioural assessment tests were performed in weeks 4-7 after initiation of dosing with the bacteria (animals 11-14 weeks old). Specifically, social behaviour and elevated plus maze tests were performed in week 5, novel object recognition tests were performed in week 6, and forced swim tests were performed in week 7.

### Results

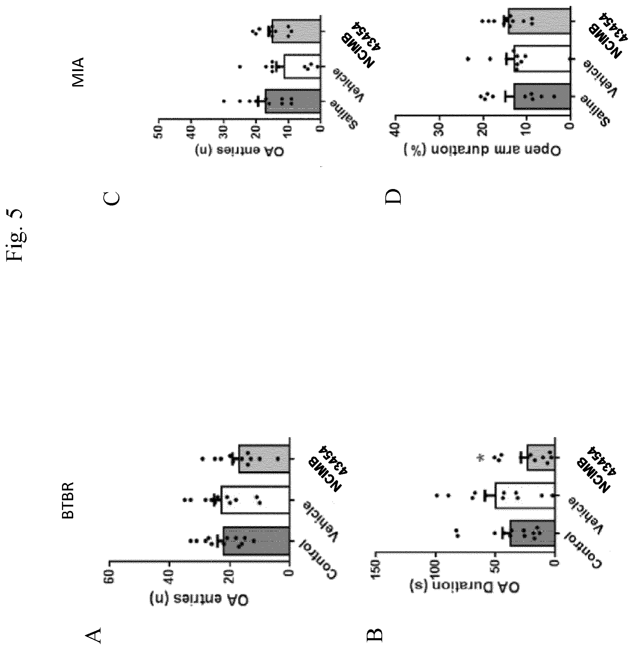

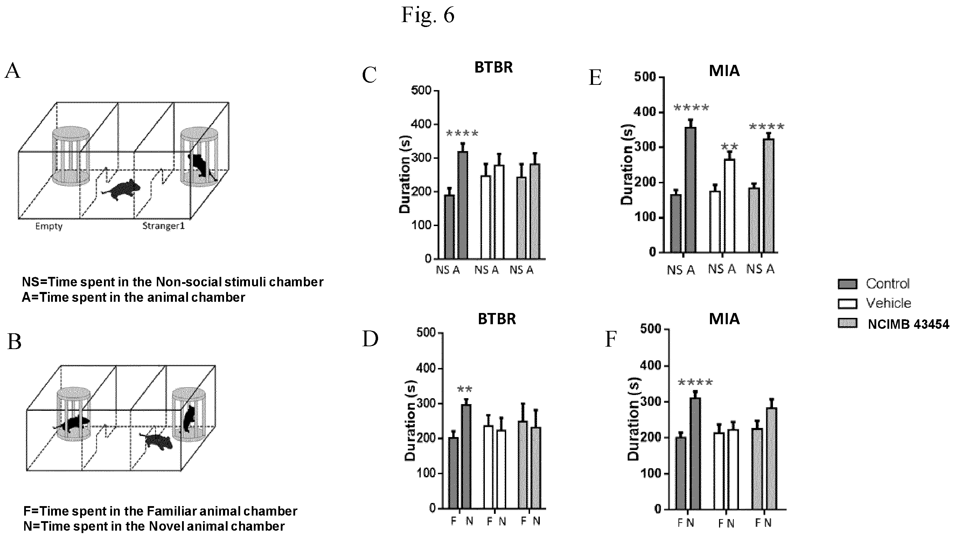

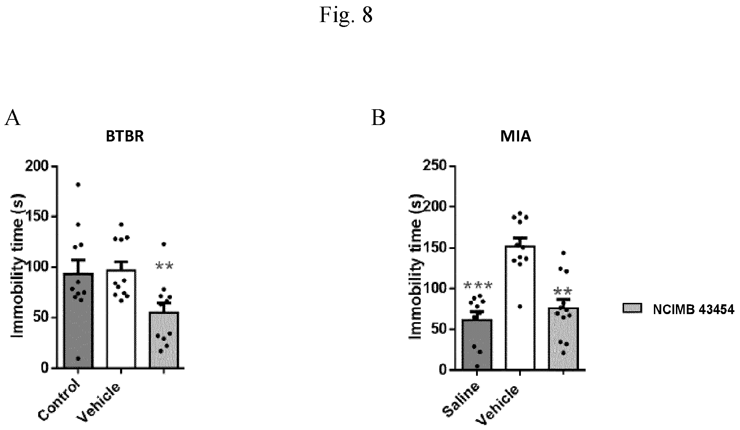

As shown in Figure 5, Btbr mice treated with *Clostridiaceae* strain NCIMB 43454 spent less time in the open arm relative to mice administered vehicle (Fig. 5B). Figure 6 shows that treatment with the bacterial strain also resulted in more social behaviour, as the MIA mice spent significantly more time in the animal chamber than the non-social stimuli chamber (Fig. 6E). Treatment with *Clostridiaceae* strain NCIMB 43454 also had a positive effect on cognitive performance, as Btbr mice spent significantly more time interacting with the novel object than the familiar object, as shown in Figure 8B. Finally, treatment with *Clostridiaceae* strain NCIMB 43454 also reduced depressive-like behaviour, as both Btbr and MIA mice spent significantly less time being immobile (Fig. 8). Together, the results indicate that *Clostridiaceae* may be useful in therapy, e.g. in the treatment or prevention CNS disorders, such as autism, anxiety and depression.

### Example 6 - Effect of Clostridiaceae on brainstem monoamine levels in models of autism spectrum disorders

### Summary

This study investigated the effect of *Clostridiaceae* strain NCIMB 43454 on brainstem monoamine levels in Btbr and MIA mouse models of autism spectrum disorder. The brainstem encompasses a number of cell bodies for all the major neurotransmitter systems of the brain including serotonin and noradrenaline.

### Materials and Methods

### Mouse models

The Btbr and MIA mouse models were generated and maintained as described in Example 5.

### Method

Animals received daily oral gavage of PBS or the live biotherapeutics prepared to 1 x 10⁹ CFU/mL in PBS. After 14 weeks, the animals were sacrificed by decapitation and the neurotransmitter concentration was analysed by HPLC on samples from the brainstem. Briefly, brainstem tissue was sonicated in 500 µl of chilled mobile phase spiked with 4 ng/40 µl of N-Methyl 5-HT (Sigma Chemical Co., UK) as internal standard. The mobile phase contained 0.1 M citric acid, 5.6 mM octane-1-sulphonic acid (Sigma), 0.1 M sodium dihydrogen phosphate, 0.01 mM EDTA (Alkem/Reagecon, Cork) and 9% (v/v) methanol (Alkem/Reagecon), and was adjusted to pH 2.8 using 4 N sodium hydroxide (Alkem/Reagecon). Homogenates were then centrifuged for 15 min at 22,000 × g at 4 °C and 40 µl of the supernatant injected onto the HPLC system which consisted of a SCL 10-Avp system controller, LECD 6A electrochemical detector (Shimadzu), a LC-10AS pump, a CTO-10A oven, a SIL-10A autoinjector (with sample cooler maintained at 40 C) and an online Gastorr Degasser (ISS, UK). A reverse-phase column (Kinetex 2.6 u C18 100 × 4.6 mm, Phenomenex) maintained at 30 °C was employed in the separation (Flow rate 0.9 ml/min). The glassy carbon working electrode combined with an Ag/AgCl reference electrode (Shimdazu) operated a +0.8 V and the chromatograms generated were analyzed using Class-VP 5 software (Shimadzu). The neurotransmitters were identified by their characteristic retention times as determined by standard injections, which run at regular intervals during the sample analysis. The ratios of peak heights of analyte versus internal standard were measured and compared with standard injection. Results were expressed as ng of neurotransmitter per g fresh weight of tissue.

### Results

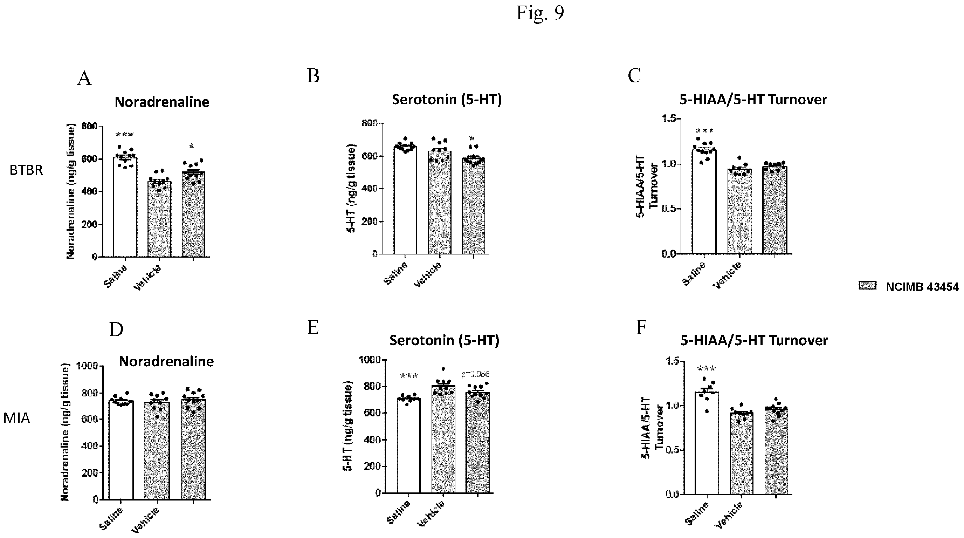

As shown in Fig. 9, mice administered *Clostridiaceae* strain NCIMB 43454 had altered levels of certain monoamines in the brainstem. Btbr mice had significantly higher noradrenaline and lower serotonin levels (Fig. 9A and B, respectively) compared with animals administered vehicle, and there were reduced serotonin levels in MIA mice (Fig. 9E). This suggests that *Clostridiaceae* is useful in the treatment or prevention of disorders associated with dysregulation of these neurotransmitter systems, e.g. CNS disorders such as autism.

### Example 7 - Effect of Clostridiaceae on gene expression in the amygdala in models of autism spectrum disorders

### Summary

This study investigated the effect of *Clostridiaceae* strain NCIMB 43454 on the expression of certain genes of interest in the brain of Btbr and MIA mice. mRNA levels for markers for the oxytocinergic system (oxytocin receptor and vasopressin receptor), endocrine system (mineralocorticoid (Nr3c1); glucocorticoid receptor (Nr3c2); corticosterone releasing factor (CRF) and receptors; Brain derived neurotrophic factor (BDNF)), immune system (Il-6, TNF-α, TLR-4); and neurotransmitter systems (NMDA receptor 2A (Grin2A); NMDA receptor 2B (Grin2B); GABAA receptor subunit A2; GABAB receptor subunit B1; serotonin 2C) were assessed in the amygdala.

### Materials and Methods

### Mouse models

The Btbr and MIA mouse models were generated and maintained as described in Example 5.

### Method

Animals received daily oral gavage of PBS or the live biotherapeutics prepared at 1 x 10⁹ CFU/mL in PBS. After 14 weeks, the animals were sacrificed by decapitation. The brain was quickly excised, dissected and each brain region was snap-frozen on dry ice and stored at -80 °C for further analysis. mRNA expression was quantified as described in Example 2.

### Results

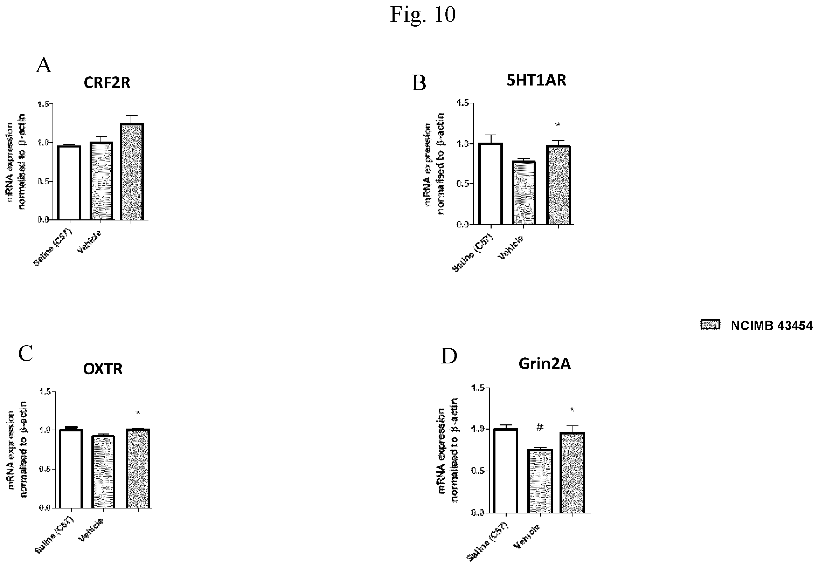

As shown in Figure 10A, Btbr mice administered *Clostridiaceae* exhibited elevated levels of CRF2R in the amygdala. Treatment with the bacteria was also associated with a significant increase in the levels of expression of 5HTAR, OXTR, Grin2a (Fig. 10B, C and D, respectively) in the amygdala of MIA mice, relative to mice administered with saline. The results indicate that this bacterial strain is useful in the treatment or prevention of disorders or conditions that may benefit from the modulation in the levels of expression of these proteins in the brain, e.g. CNS diseases and disorders.

### SEQUENCES

SEQ ID NO: 1 - Clostridiaceae strain deposited under accession number NCIMB 43454 16S
ribosomal RNA
CTTCGGCCGCGCCCCTTTGCAGTTAGGCTACGGACTTCGGGTGTTCCCGACTCTCATGGTGTGA
CGGGCGGTGTGTACAAGGCCCGGGAACGTATTCACCGCGGCATGCTGATCCGCGATTACTAGCG
ATTCCGACTTCATACAGGCGGGTTTCAGCCTGCAATCCGAACTGGGACGGCTTTTAGGGGTTTG
CTCTGCCTCGCGGCTTTGCCTCCCTCTGTTAACCGCCATTGTAGTACGTGTGTGGCCCAGGACA
TAAGGGGCATGATGATTTGACGTCGTCCCCACCTTCCTCCGTTTTGTCAACGGCAGTCTCGCTA
GAGTGCTCTTGCGTAGCAACTAACAATAAGGGTTGCGCTCGTTGCGGGACTTAACCCAACATCT
CACGACACGAGCTGACGACAACCATGCACCACCTGTCTCTACTTTCCCCGAAGGGCACCTAATC
CATCTCTGGTTCGTTAGTAGGATGTCAAGCCCTGGTAAGGTTCTTCGCGTTGCTTCGAATTAAA
CCACATACTCCACCGCTTGTGCGGGCCCCCGTCAATTCCTTTGAGTTTCAACCTTGCGATCGTA
CTCCCCAGGTGGGATACTTATTGCGTTAGCTGCGGCACGGAGGGGGTCAGACCCCCACACCTA
GTATCCATCGTTTACGGCGTGGACTACCAGGGTATCTAATCCTGTTTGCTCCCCACGCTTTCGC
GCCTCAGCGTCAGTTATCGTCCAGCAATCCGCCTTCGCCACTGGTGTTCCTCCGTATATCTACG
CATTTCACCGCTACACACGGAATTCCGATTGCCTCTCCGACACTCAAGAACTACAGTTTCAAAT
GCAGGCTATGGGTTGAGCCCATAGTTTTCACATCTGACTTGCAGTCCCGCCTACACGCCCTTTA
CACCCAGTAAATCCGGATAACGCTTGCCACCTACGTATTACCGCGGCTGCTGGCACGTAGTTAG
CCGTGGCTTATTCAACAGGTACCGTCACTTGCTTCGTCCCTGTTAAAAGAAGTTTACAACCCGA
AAGCCTTCTTCCTTCACGCGGCGTTGCTGGGTCAGGCTTGCGCCCATTGCCCAATATTCCCCAC
TGCTGCCTCCCGTAGGAGTCTGGGCCGTGTCTCAGTCCCAATGTGGCCGGCCAACCTCTCAGTC
CGGCTACTGATCGTCGACTTGGTGGGCCGTTACCCCGCCAACTATCTAATCAGACGCGAGTCCA
TCTCAGAGCGATAAATCTTTGATATCAGTGCCATGCGACACCAATATGTCATGCGGTATTAGCA
CAGTTTTCACTGTGTTATTCCCCACTCCAAGGCAGGTTACTCACGCGTTACTCACCCGTCCGCC
ACTAAACAGCCAATTCCATTGTCCGAAAACTCTTTCCTTGGCGTCCGTCGACTGC

### REFERENCES

[1] Spor et al. (2011) Nat Rev Microbiol. 9(4):279-90.
[2] Eckburg et al. (2005) Science. 10;308(5728):1635-8.
[3] Macpherson et al. (2001) Microbes Infect. 3(12):1021-35
[4] Macpherson et al. (2002) Cell Mol Life Sci. 59(12):2088-96.
[5] Mazmanian et al. (2005) Cell 15;122(1):107-18.
[6] Frank et al. (2007) PNAS 104(34):13780-5.
[7] Scanlan et al. (2006) J Clin Microbiol. 44(11):3980-8.
[8] Kang et al. (2010) Inflamm Bowel Dis. 16(12):2034-42.
[9] Machiels et al. (2013) Gut. 63(8):1275-83.
[10] Mayer et al (2014) The Journal of Neuroscience 34(46):15490 -15496
[11] Cryan and Dinan (2015) Neuropsychopharmacology, 40: 241-2.
[12] Zhou and Foster (2015) Neuropsychiatric Disease and Treatment 11: 715-723.
[13] Wang and Kasper (2014) Brain Behav Immun. 38: 1-12.
[14] WO 2013/050792
[15] WO 03/046580
[16] WO 2013/008039
[17] WO 2014/167338
[18] Goldin and Gorbach (2008) Clin Infect Dis. 46 Suppl 2:596-100.
[19] Azad et al. (2013) BMJ. 347:f6471.
[20] Bravo et al. (2011) Proc Natl Acad Sci U S A, 108: 16050-5.
[21] Kantak et al. (2014) Behav Pharmacol. 25: 71-9.
[22] Savignac et al. (2014) Neurogastroenterol Motil. 26: 1615-27.
[23] de Theije et al. (2014) Brain Behav Immun. 37: 197-206.
[24] Hsiao et al. (2013) Cell, 155: 1451-63.
[25] Meyza and Blanchard (2017) Neurosci Biobehav Rev.
[26] Dürre Microbiol Spectr. 2014 Aug;2(4)
[27] Chun et al (2007). Int J Syst Evol Microbiol 57: 2259-2261.
[28] Masco et al. (2003) Systematic and Applied Microbiology, 26:557-563.
[29] Srůtková et al. (2011) J. Microbiol. Methods, 87(1):10-6.
[30] Wang et al. (2016) J Neurogastroenterol Motil 22: 589-605.
[31] Li and Zhou (2016) Neuroscience 324: 131-139.
[32] Tadano et al. (2004) Methods Find Exp Clin Pharmacol 26:93-7.
[33] Andero et al. (2014) Prog Mol Biol Transl Sci. 122:169-92.
[34] Philips et al. (1991) Neuron. 7:695-702.
[35] Ng et al. (2019) Int J Mol Sci. 20: pii: E257.
[36] Ferrer et al. (2000) Brain Res. 866:257-61.
[37] Delaville et al. (2011) Front Syst Neurosci. 5: 31.
[38] Zhou (2004) Drugs Future. 29: 1235-1244.
[39] Muller et al. (2016) Neuroscience. 321: 24-41.
[40] Miyamoto-Shinohara et al. (2008) J. Gen. Appl. Microbiol., 54, 9-24.
[41] Cryopreservation and Freeze-Drying Protocols, ed. by Day and McLellan, Humana Press.
[42] Leslie et al. (1995) Appl. Environ. Microbiol. 61, 3592-3597.
[43] Mitropoulou et al. (2013) J Nutr Metab. (2013) 716861.
[44] Kailasapathy et al. (2002) Curr Issues Intest Microbiol. 3(2):39-48.
*[45]* Handbook of Pharmaceutical Excipients, 2nd Edition, (1994), Edited by A Wade and PJ Weller
*[46]* Remington's Pharmaceutical Sciences, Mack Publishing Co. (A. R. Gennaro edit. 1985)
[47] US 2016/0067188
*[48]* Handbook of Microbiological Media, Fourth Edition (2010) Ronald Atlas, CRC Press.
*[49]* Maintaining Cultures for Biotechnology and Industry (1996) Jennie C. Hunter-Cevera, Academic Press
[50] Strobel (2009) Methods Mol Biol. 581:247-61.
[51] Gennaro (2000) Remington: The Science and Practice of Pharmacy. 20th edition, ISBN: 0683306472.
*[52]* Molecular Biology Techniques: An Intensive Laboratory Course, (Ream et al., eds., 1998, Academic Press).
[53] Methods In Enzymology (S. Colowick and N. Kaplan, eds., Academic Press, Inc.)
*[54]* Handbook of Experimental Immunology, Vols. I-IV (D.M. Weir and C.C. Blackwell, eds, 1986, Blackwell Scientific Publications)
[55] Sambrook et al. (2001) Molecular Cloning: A Laboratory Manual, 3rd edition (Cold Spring Harbor Laboratory Press).
[56] Handbook of Surface and Colloidal Chemistry (Birdi, K.S. ed., CRC Press, 1997)
[57] Ausubel et al. (eds) (2002) Short protocols in molecular biology, 5th edition (Current Protocols).
[58] PCR (Introduction to Biotechniques Series), 2nd ed. (Newton & Graham eds., 1997, Springer Verlag)
[59] Current Protocols in Molecular Biology (F.M. Ausubel et al., eds., 1987) Supplement 30
[60] Smith & Waterman (1981) Adv. Appl. Math. 2: 482-489.

## Claims

1. A composition comprising a bacterial strain of the family *Clostridiaceae* for use in a method of treating or preventing a central nervous system disorder or condition.

2. The composition for use of claim 1, wherein the central nervous system disorder or condition is mediated by the microbiota-gut-brain axis.

3. The composition for use of any one of the preceding claims, wherein the composition is for use in a method of treating or preventing a disorder or condition selected from the group consisting of: autism spectrum disorders (ASDs); child developmental disorder; obsessive compulsive disorder (OCD); major depressive disorder; depression; seasonal affective disorder; anxiety disorders; chronic fatigue syndrome (myalgic encephalomyelitis); stress disorder; post-traumatic stress disorder; schizophrenia spectrum disorders; schizophrenia; bipolar disorder; psychosis; mood disorder; dementia; Alzheimer's; Parkinson's disease; chronic pain; motor neuron disease; Huntington's disease; Guillain-Barre syndrome and meningitis.

4. The composition for use of claim 3, wherein the disorder or condition is autism spectrum disorder.

5. The composition for use of claim 4, wherein the composition is for use in a method of reducing or preventing autism.

6. The composition for use of any one of the preceding claims, wherein the bacterial strain is the strain deposited under accession number NCIMB 43454.

7. The composition for use of any one of the preceding claims, wherein the bacterial strain has a 16S rRNA sequence that is at least 95%, 96%, 97%, 98%, 99%, 99.5% or 99.9% identical to SEQ ID NO: 1.

8. The composition for use of any one of the preceding claims, wherein the bacterial strain has a 16S rRNA sequence that is at least 95%, 96%, 97%, 98%, 99%, 99.5% or 99.9% identical to the 16S rRNA sequence of a bacterial strain of *Clostridiaceae.*

9. A composition comprising a bacterial strain, wherein the bacterial strain has a 16S rRNA sequence that is at least 95%, 96%, 97%, 98%, 99%, 99.5% or 99.9% identical to SEQ ID NO: 1 or wherein the bacterial strain has the 16S rRNA sequence represented by SEQ ID NO: 1, for use in therapy.

10. A composition comprising a bacterial strain, wherein the bacterial strain has a 16S rRNA sequence that is least 95%, 96%, 97%, 98%, 99%, 99.5% or 99.9% identical to SEQ ID NO: 1 or wherein the bacterial strain has the 16S rRNA sequence represented by SEQ ID NO: 1, for use in a method of treating or preventing a central nervous system disorder or condition.

11. The composition for use of any one of the preceding claims, wherein the composition is for oral administration.

12. The composition for use of any one of the preceding claims, wherein the composition comprises one or more pharmaceutically acceptable excipients or carriers.

13. The composition for use of any one of the preceding claims, wherein the bacterial strain is lyophilised.

14. The composition for use of any one of the preceding claims, wherein the bacterial strain is viable and capable of partially or totally colonising the intestine.

15. The composition for the use of any preceding claim, wherein the bacterial strain has at least 95%, 96%, 97%, 98%, 99%, 99.5% or 99.9% identity to the bacterial strain deposited under NCIMB 43454.
